# FASCICULE DE BREVET EUROPEEN

(11) **EP 1 385 980 B1**
(45) Date de publication et mention de la délivrance du brevet: **27.10.2010**
(21) Numéro de dépôt: 02708406.0
(22) Date de dépôt: 29.01.2002
(51) Int. Cl.: C12P 33/00, C12P 33/08, C12P 33/18

(54) **SOUCHES DE LEVURES PRODUISANT DES STEROIDES DE FACON AUTONOME**
HEFE STÄMME DIE AUTONOM STEROIDE PRODUZIEREN
YEAST STRAINS AUTONOMOUSLY PRODUCING STEROIDS

(30) Priorité: 31.01.2001 FR 0101294
(43) Date de publication de la demande: 04.02.2004
(73) Titulaire: Aventis Pharma S.A., 92160 Antony (FR)
(72) Inventeur: SPAGNOLI, Roberto, F-75016 PARIS (FR); ACHSTETTER, Tilman, 28213 BREMEN (DE); CAUET, Gilles, F-67370 GRIESHEIM-SUR-SOUFFEL (FR); DEGRYSE, Eric, F-91560 CROSNE (FR); DUMAS, Bruno, F-78117 CHATEAUFORT (FR); POMPON, Denis, F-91190 GIF-SUR-YVETTE (FR); WINTER, Jacques, F-77860 QUINCY-VOISINS (FR)
(74) Mandataire: Bouvet, Philippe
(86) Numéro de dépôt international: PCT/FR2002/000348
(87) Numéro de publication internationale: WO 2002/061109

(56) Documents cités:
- EP-A- 0 360 361
- EP-A- 0 727 489
- WO-A-99/40203
- US-A- 5 137 822
- DEGRYSE E ET AL: "Pregnenolone metabolized to 17alpha-hydroxyprogesterone in yeast: Biochemical analysis of a metabolic pathway." JOURNAL OF STEROID BIOCHEMISTRY AND MOLECULAR BIOLOGY., vol. 71, no. 5-6, 31 décembre 1999 (1999-12-31), pages 239-246, XP001024829 ISSN: 0960-0760 cité dans la demande
- DUPORT C ET AL: "Self-sufficient biosynthesis of pregnenolone and progesterone in engineered yeast" NATURE BIOTECHNOLOGY, vol. 16, no. 2, 1 février 1998 (1998-02-01), pages 186-189, XP002084824 ISSN: 1087-0156 cité dans la demande
- DUMAS B ET AL: "11BETA-HYDROXYLASE ACTIVITY IN RECOMBINANT YEAST MITOCHONDRIA IN VIVO CONVERSION OF 11-DEOXYCORTISOL TO HYDROCORTISONE" EUROPEAN JOURNAL OF BIOCHEMISTRY, vol. 238, 1996, pages 495-504, XP000992874 ISSN: 0014-2956 cité dans la demande
- LI JIE ET AL: "Adrenodoxin reductase homolog (Arh1p) of yeast mitochondria required for iron homeostasis." JOURNAL OF BIOLOGICAL CHEMISTRY, vol. 276, no. 2, 12 janvier 2001 (2001-01-12), pages 1503-1509, XP002238717 ISSN: 0021-9258
- LACOUR, T. ET AL.: "Characterization of recombinant adrenodoxin reductase homologue (Arh1p) from yeast: Implication in in vitro cytochrome P45011beta monooxygenase system" JOURNAL OF BIOLOGICAL CHEMISTRY, vol. 273, no. 37, pages 23984-23992, XP002238718 ISSN: 0021-9258 cité dans la demande
- YANG HONGYUAN ET AL: "Sterol esterification in yeast: A two-gene process." SCIENCE (WASHINGTON D C), vol. 272, no. 5266, 1996, pages 1353-1356, XP002179474 ISSN: 0036-8075
- SKAGGS B A ET AL: "Cloning and characterization of the Saccharomyces cerevisiae C-22 sterol desaturase gene, encoding a second cytochrome P-450 involved in ergosterol biosynthesis" GENE, vol. 169, no. 1, 22 février 1996 (1996-02-22), pages 105-109, XP004042996 ISSN: 0378-1119
- CAUET G ET AL: "CYP11A1 stimulates the hydroxylase activity of CYP11B1 in mitochondria of recombinant yeast in vivo and in vitro." EUROPEAN JOURNAL OF BIOCHEMISTRY, (2001 JUL) 268 (14) 4054-62., XP002179473
- SZCZEBARA FLORENCE MENARD ET AL: "Total biosynthesis of hydrocortisone from a simple carbon source in yeast." NATURE BIOTECHNOLOGY, vol. 21, no. 2, 20 février 2003 (2003-02-20), pages 143-149, XP002238719 ISSN: 1087-0156

### LISTE DE SEQUENCES

<110> AVENTIS PHARMA SA
<120> Souche de levure produisant des stéroïdes de façon autonome
<130> Demande RMV 2544
<140>
   <141>
<150> FR 0101294
   <151> 2001-01-31
<160> 49
<170> PatentIn Ver. 2.1
<210> 1
   <211> 32
   <212> ADN
   <213> Séquence artificielle
<220>
   <223> Oligonucléotide OTG11314 pour amplifier la partie 5' de YPR1
<400> 1
   tacgctcgag acgttggtgt cattgatatt ca 32
<210> 2
   <211> 21
   <212> ADN
   <213> Séquence artificielle
<220>
   <223> Oligonucléotide OTG11315 pour amplifier la partie 5' de YPR1
<400> 2
   cttcattcaa atagatagcc g 21
<210> 3
   <211> 22
   <212> ADN
   <213> Séquence artificielle
<220>
   <223> Oligonucléotide OTG11316 pour amplifier la partie 3' de YPR1
<400> 3
   tatggctaaa aagcacggcg tt 22
<210> 4
   <211> 31
   <212> ADN
   <213> Séquence artificielle
<220>
   <223> Oligonucléotide OTG11317 pour amplifier la partie 3' de YPR1
<400> 4
   cgatctcgag tttctcgttg ttcaggtact g 31
<210> 5
   <211> 65
   <212> ADN
   <213> Séquence artificielle
<220>
   <223> Oligonucléotide OTG11463 pour amplifier URA3 flanqué de YPR1 <400> 5
<210> 6
   <211> 58
   <212> ADN
   <213> Séquence artificielle
<220>
   <223> Oligonucléotide OTG11464 pour amplifier URA3 flanqué de YPR1
<400> 6
   aacgccgtgc tttttagcca taagcttggg taataactga tataattaaa tagtactc 58
<210> 7
   <211> 40
   <212> ADN
   <213> Séquence artificielle
<220>
   <223> Oligonucléotide OTG7410 pour amplifier l'ADNc de la P450c21 humaine
<400> 7
   ggaattccgt cgacaaaaat gctgctcctg ggcctgctgc 40
<210> 8
   <211> 29
   <212> ADN
   <213> Séquence artificielle
<220>
   <223> Oligonucléotide OTG5927 pour amplifier l'ADNc de la P450c21 humaine
<400> 8
   cctcaatggt cctcttggag ttcagcacc 29
<210> 9
   <211> 32
   <212> ADN
   <213> Séquence artificielle
<220>
   <223> Environnement autour du codon ATG d'initiation de la traduction de P450c21
<400> 9
   gtcgacaaaa atgctgctcc tgggcctgct gc 32
<210> 10
   <211> 23 <212> ADN
   <213> Séquence artificielle
<220>
   <223> Oligonucléotide OTG11844
<400> 10
   tttgctcgag gttacagaag ggc 23
<210> 11
   <211> 24
   <212> ADN
   <213> Séquence artificielle
<220>
   <223> Oligonucléotide OTG11845
<400> 11
   gattctcgag caattggctg acta 24
<210> 12
   <211> 14
   <212> ADN
   <213> Séquence artificielle
<220>
   <223> Oligonucléotide ATG11975
<400> 12
   aaatcgataa catg 14
<210> 13
   <211> 14
   <212> ADN
   <213> Séquence artificielle
<220>
   <223> Oligonucléotide OTG11976
<400> 13
   ttatcgattt catg 14
<210> 14
   <211> 30
   <212> ADN
   <213> Séquence artificielle
<220>
   <223> Oligonucléotide OTG11981
<400> 14
   attgatatcg ataaaaagca cggcgttgag 30
<210> 15
   <211> 26
   <212> ADN
   <213> Séquence artificielle
<220>
   <223> Oligonucléotide OTG11982
<400> 15
   tctcggaatt caggtactgc agccag 26
<210> 16
   <211> 30
   <212> ADN
   <213> Séquence artificielle
<220>
   <223> Oligonucléotide OTG11980
<400> 16
   caactaagct tcattcaaat agatagccgc 30
<210> 17
   <211> 50
   <212> ADN
   <213> Séquence artificielle
<220>
   <223> Oligonucléotide OTG11285
<400> 17
   gattcggtaa tctccgaaca ggtaccaatt atatcagtta ttacccggga 50
<210> 18
   <211> 19
   <212> ADN
   <213> Séquence artificielle
<220>
   <223> Oligonucléotide OTG11286
<400> 18
   agccatcttc aaagcggtt 19
<210> 19
   <211> 22
   <212> ADN
   <213> Séquence artificielle
<220>
   <223> Oligonucléotide OTG11287
<400> 19
   ccgatcgaat caaaacgaac ag 22
<210> 20
   <211> 20
   <212> ADN
   <213> Séquence artificielle
<220>
   <223> Oligonucléotide OTG11289
<400> 20
   tctaatcagc tagtaagaac 20
<210> 21
   <211> 67
   <212> ADN
   <213> Séquence artificielle
<220>
   <223> Oligonucléotide OTG11305
<400> 21
   aaccgctttg aaagatggct atcgattttc aattcaattc atcatttttt ttttattctt 60
   ttttttg 67
<210> 22
   <211> 60
   <212> ADN
   <213> Séquence artificielle
<220>
   <223> Oligonucléotide OTG11306
<400> 22
   ctgttcgttt tgattcgatc gggaagcttg ggtaataact gatataatta aattgaactc 60
<210> 23
   <211> 138
   <212> PRT
   <213> Séquence artificielle
<220>
   <223> Séquence N-Terminal de la protéine construite pour l'expression de la P450c11
<400> 23
   Met Leu Ser Arg Ala Ile Phe Arg Asn Pro Val Ile Asn Arg Thr Leu
   1 5 10 15
   Leu Arg Ala Arg Pro Gly Ala Tyr His Ala Thr Arg Leu Thr Lys Asn
   20 25 30
   Thr Phe Ile Gln Ser Arg Lys Tyr Gly Thr Arg Gly Ala Ala Ala Pro
   35 40 45
   Lys Ala Val Leu Pro Phe Glu Ala Met Pro Arg Cys Pro Gly Asn Lys
   50 55 60
   Trp Met Arg Met Leu Gln Ile Trp Arg Glu Gln Gly Tyr Glu Asp Leu
   65 70 75 80
   His Leu Glu Val His Gln Thr Phe Gln Glu Leu Gly Pro Ile Phe Arg
   85 90 95
   Tyr Asp Leu Gly Gly Ala Gly Met Val Cys Val Met Leu Pro Glu Asp
   100 105 110
   Val Glu Lys Leu Gln Gln Val Asp Ser Leu His Pro His Arg Met Ser
   115 120 125
   Leu Glu Pro Trp Val Ala Tyr Arg Gln His
   130 135
<210> 24
   <211> 37
   <212> ADN
   <213> Séquence artificielle
<220>
   <223> Oligonucléotide OLPI FL
<400> 24
   agctggcggc cgcttaatta agtggcgcgc caagctt 37
<210> 25 <211> 13
   <212> ADN
   <213> Séquence artificielle
<220>
   <223> Séquence rajoutée devant la 3 beta-HSDH bovine
<400> 25
   gtcgacaaaa atg 13
<210> 26
   <211> 24
   <212> ADN
   <213> Séquence artificielle
<220>
   <223> Fin de l'ADNc de la 3 beta-HSDH bovine
<400> 26
   tgacctggag tgacaatgac gcgt 24
<210> 27
   <211> 9
   <212> ADN
   <213> Séquence artificielle
<220>
   <223> Oligonucléotide OTG5868
<400> 27
   cgcgtgtac 9
<210> 28
   <211> 28
   <212> ADN
   <213> Séquence artificielle
<220>
   <223> Oligonucléotide OLIP 174
<400> 28
   agctgcggcc gcggcgcgcc gtttaaac 28
<210> 29
   <211> 28
   <212> ADN
   <213> Séquence artificielle
<220>
   <223> Oligonucléotide OLIP 175
<400> 29
   agctgtttaa acggcgcgcc gcggccgc 28
<210> 30
   <211> 27
   <212> ADN
   <213> Séquence artificielle
<220>
   <223> Oligonucléotide OLIP 21
<400> 30
   aaacggcgca gtagggaata ttactgg 27
<210> 31
   <211> 30
   <212> ADN
   <213> Séquence artificielle
<220>
   <223> Oligonucléotide OLIP 22
<400> 31
   ccgaagctta atcggcaaaa aaagaaaagc 30
<210> 32
   <211> 12
   <212> ADN
   <213> Séquence artificielle
<220>
   <223> Oligonucléotide OLIP 20
<400> 32
   agctgcggcc gc 12
<210> 33
   <211> 22
   <212> ADN
   <213> Séquence artificielle
<220>
   <223> Oligonucléotide C17-3
<400> 33
   actgatggtt ggggtgcatt ga 22
<210> 34
   <211> 22
   <212> ADN
   <213> Séquence artificielle
<220>
   <223> Oligonucléotide C17-5
<400> 34
   atggcatcct ggaggttctg ag 22
<210> 35
   <211> 23
   <212> ADN
   <213> Séquence artificielle
<220>
   <223> Oligonucléotide ADX-3
<400> 35
   gtacccgggg atccttattc tat 23
<210> 36
   <211> 24
   <212> ADN
   <213> Séquence artificielle
<220>
   <223> Oligonucléotide ADX-5
<400> 36
   cagtccactt tataaaccgt gatg 24
<210> 37
   <211> 39
   <212> ADN
   <213> Séquence artificielle
<220>
   <223> Oligonucléotide 5'ADE2090
<400> 37
   cgattcggat ccactagtaa cgccgtatcg tgattaacg 39
<210> 38
   <211> 40
   <212> ADN
   <213> Séquence artificielle
<220>
   <223> Oligonucléotide 3'ADE2089
<400> 38
   cctcaaggat cctcctgacg tagcgctatc ctcggttctg 40
<210> 39
   <211> 327
   <212> ADN
   <213> Séquence artificielle
<220>
   <223> Pied de recombinaison R1
<400> 39
   atggcccttc aagctgcttt ctttggtctc ctctgctttc tctgtccgca aagatggaaa 60
   attaaatgct tcagcatcat catcattcaa agagtctagt ctgttcggtg tttcactttc 120
   ggagcaaagc aaagctgact ttgtctcttc ctcattgaga tgcaagaggg aacagagctt 180
   gaggaataat aaagcgatta ttcgagctca agcaatcgcg acttcaactc catcagtcac 240
   aaaatcttcc ttagaccgca agaaaacact tagaaaagga aacgtggttg tcacgggagc 300
   ttcttcaggg ctaggtttag caacggc 327
<210> 40
   <211> 336
   <212> ADN
   <213> Séquence artificielle
<220>
   <223> Pied de recombinaison R2
   <400> 40
   ataatggcgt gcagagactt cctcaaggct gagagagccg ctcaatctgc agggatgcct 60
   aaggacagct acactatgat gcatttggac ttggcgtctt tggacagcgt gaggcagttt 120
   gttgataact tcaggcgagc tgagatgcct ctcgatgtgt tggtctgcaa tgccgcagtc 180
   tatcagccaa cggctaatca acctactttc actgctgaag ggtttgagct tagcgttggg 240
   ataaaccatt tgggccactt tcttctttca agattgttga ttgatgactt gaagaactcc 300
   gattatccat caaaacgtct catcattgtt ggtacc 336
<210> 41
   <211> 46
   <212> ADN
   <213> Séquence artificielle
<220>
   <223> Oligonucléotide OTG10842
<400> 41
   aaaaacgcgt aactattaaa gcgacgcaaa ttcgccgatg gtttgg 46
<210> 42
   <211> 50
   <212> ADN
   <213> Séquence artificielle
<220>
   <223> Oligonucléotide OTG1084
<400> 42
   aaaagtcgac aaaatggaag atatagaagg atacgaacca catatcactc 50
<210> 43
   <211> 20
   <212> ADN
   <213> Séquence artificielle
<220>
   <223> Oligonucléotide arh1D
<400> 43
   cggagcgcta tccttagaga 20
<210> 44
   <211> 20
   <212> ADN
   <213> Séquence artificielle
<220>
   <223> Oligonucléotide nfs1R
<400> 44
   acgtttcttt cgcctacgtg 20
<210> 45
   <211> 20
   <212> ADN
   <213> Séquence artificielle
<220>
   <223> Oligonucléotide leu2D
<400> 45
   ggtaaggcca ttgaagatgc 20
<210> 46
   <211> 20
   <212> ADN
   <213> Séquence artificielle
<220>
   <223> Oligonucléotide arh1R
<400> 46
   accccttttg ttccgagttc 20
<210> 47
   <211> 20
   <212> ADN
   <213> Séquence artificielle
<220>
   <223> Oligonucléotide X1TEF1
<400> 47
   ttcaaaacac ccaagcacag 20
<210> 48
   <211> 20
   <212> ADN
   <213> Séquence artificielle
<220>
   <223> Oligonucléotide X2C21
<400> 48
   ggtctgccag caaagtctgc 20
<210> 49
   <211> 20
   <212> ADN
   <213> Séquence artificielle
<220>
   <223> Oligonucléotide X3TDH3
<400> 49
   cgggcacaac ctcaatggag 20

## Description

La présente invention concerne la production de stéroïdes dans les microorganismes, notamment les souches de levures.

Les stéroïdes, notamment les stéroïdes dérivés du cholestérol, sont impliqués dans de nombreux processus physiologiques parmi lesquels on peut citer la régulation des taux de glucides et cholestérol dans la circulation sanguine, le maintien et développement de la masse musculaire, le développement du système nerveux central.

Parmi les inconvénients observés en cas de dérèglement des taux de stéroïdes circulants, on peut citer le déclenchement éventuel de maladies auto-immunes, telles le lupus, de certains cancers, par exemple le cancer du sein, de maladies cardiovasculaires, par exemple l'athérosclérose. Des problèmes de régulation de stéroïdes sont également soupçonnés dans le cas du déclenchement de certaines maladies neurologiques tels les maladies de Parkinson ou d'Alzheimer.

Les stéroïdes, notamment l'hydrocortisone, peuvent être utilisés en tant qu'agents thérapeutiques en tant que tel ou comme compléments dans d'autres traitements. Ainsi, les dérivés de synthèse des glucocorticoïdes sont utilisés pour leurs actions anti-inflammatoires et, à fortes doses, immunosuppressives.

L'obtention des stéroïdes est actuellement liée à des procédés d'extraction ou de synthèse coûteux. John Warcup Cornforth a été le premier à réaliser la synthèse totale d'un stéroïde, le cholestérol, par une méthode enzymatique. Toutefois, il est important de disposer d'une méthode permettant d'obtenir les stéroïdes d'intérêt, notamment les dérivés du cholestérol, à un prix abordable.

Un certain nombre de protéines impliquées dans la biosynthèse des stéroïdes ont été exprimées chez la levure. Ainsi, le brevet EP 360 361 démontre l'activité des protéines P450 17α et P450c21 chez la levure *Kluyveromyces lactis*. De même, il a été décrit la possibilité de conversion *in vivo* de 11 deoxycorfiisol en hydrocortisone dans une levure génétiquement modifiée exprimant la P450c11 (Dumas *et al.,* 1996), ainsi que la production de 17α-hydroxyprogestérone à partir de prégnénolone chez la levure (Degryse *et al.,* 1999). De plus Duport *et al*. (Duport *et al.,* 1998) ont décrit la synthèse de prégnénolone et de progestérone dans une levure génétiquement modifiée. Il a également été décrit dans la demande de brevet WO 99/40203 que l'inactivation du gène *ATF2* dans une souche de levure permet d'éviter l'acétylation des stéroïdes produits par cette souche.

La présente invention permet de réaliser la synthèse de stéroïdes, par la fermentation de souches de levures génétiquement modifiées, en présence d'une source de carbone simple. La méthode proposée par la présente invention permet donc d'obtenir une grande quantité de stéroïdes d'intérêt, à faible coût, puisque le procédé met en oeuvre la fermentation de levures et l'ajout d'une source de carbone simple, facilement disponible dans le commerce.

### Définitions :

Par source de carbone simple, selon la présente invention, on entend des sources de carbone utilisables par l'homme du métier pour la croissance normale d'une levure. On entend désigner notamment les différents sucres assimilables, tels le glucose, le galactose ou le saccharose, ou les mélasses, ou les sous-produits de ces sucres. Une source de carbone simple tout particulièrement préférée est l'éthanol et le glycérol.

Par dérivé d'un stéroïde, on entend désigner, selon la présente invention, un composé pouvant être obtenu, notamment par une ou deux réactions enzymatiques ou chimiques, à partir dudit stéroïdes. On entend particulièrement désigner les stéroïdes acétylés, hydroxylés, ou portant un substituant tel qu'un dérivé halogéné (fluor, iode), ou un groupe méthyle.

Les problèmes à résoudre pour pouvoir produire des stéroïdes dans un micro-organisme sont de différentes sortes :
- il convient d'éliminer les réactions parasites pouvant être observées en raison de la présence d'enzymes endogènes dans le microorganisme choisi,
- il convient d'introduire les gènes permettant la modification des intermédiaires de synthèse de telle façon que les niveaux d'expression obtenus soient aussi proches que possible des niveaux observés chez les mammifères. Ainsi, recréer les bons équilibres est une condition importante de la réussite d'un tel projet,
- il convient d'obtenir un niveau d'expression des différents gènes permettant d'orienter préférentielleinent la biosynthèse vers le stéroïde choisi.

La synthèse des stéroïdes est une suite de réactions extrêmement complexes, mettant en jeu plusieurs enzymes pour obtenir le cortisol à partir du cholestérol.

Il convient d'abord de produire le 20,22 dihydroxycholestérol, qui est alors transformé en prégnénolone, elle-même hydroxylée en 17-α hydroxy-prégnénolone. Celle-ci est alors transformée en 17-α hydroxy-progestérone, qui donne le désoxycortisol, menant au cortisol (hydrocortisone).

Une voie alternative consiste en production de prégnénolone, puis de progestérone, transformée en 17-α hydroxy-progestérone.

Il a été montré que l'on peut produire de la prégnénolone dans la levure, à partir d'une source de carbone simple, (Duport *et al*., 1998, dont le contenu est incorporé par référence à la présente demande). Pour ce faire, il a été nécessaire de supprimer une voie endogène (par disruption du gène Δ22-stérol-désaturase (*ERG5*) de la voie de biosynthèse endogène des ergostérols), afin d'obtenir une accumulation de produits saturés en C-22. En effet, l'ergostérol normalement fabriqué par la levure diffère du cholestérol par une insaturation en C-7(8) du noyau B, une insaturation en C-22, et un groupe méthyle additionnel en C-24. Ainsi, les produits saturés en C-22, et en C-7 dans le noyau B peuvent être utilisés comme substrats par les enzymes situées dans la chaîne de production du cortisol.

La présente invention permet de réaliser la synthèse de stéroïdes, et notamment les stéroïdes situés plus en aval que la prégnénolone, de façon simple, par la fermentation de souches de levures génétiquement modifiées, en présence d'une source de carbone simple. Dans un cas particulier de l'invention, les stéroïdes synthétisés sont excrétés dans le milieu de culture, ce qui simplifie leur purification. La méthode proposée par la présente invention permet donc d'obtenir une grande quantité de stéroïdes d'intérêt, à faible coût, puisque le procédé met en oeuvre la fermentation de levures et l'ajout d'une source de carbone simple, facilement disponible dans le commerce.

De façon préférée, les stéroïdes pouvant être produits par la souche de levure selon l'invention sont des stéroïdes compris dans la voie de synthèse du cortisol, ainsi qu'énoncés ci-dessus. On peut également produire d'autres types de stéroïdes, à partir de la 17-α hydroxy-prégnénolone, notamment la dihydroépiandrostérone (DHEA), par l'action de l'enzyme 17 α-hydroxylase et lyase, et les stéroïdes dérivés (androstènediones, testostérone...). Ces stéroïdes peuvent être produits par introduction des enzymes appropriées dans la souche de levure, de la même façon que pour la souche exemplifiée dans la présente invention.

Pour la mise en oeuvre de la méthode selon l'invention, l'invention concerne également une souche de levure, génétiquement modifiée produisant un stéroïde ou un dérivé de stéroïde, **caractérisée en ce qu**'elle permet la production de manière autonome à partir d'une source de carbone simple.

Le fait que la production soit effectuée de manière autonome signifie qu'il n'est pas besoin de rajouter de substrats pour obtenir le stéroïde d'intérêt, mais que la levure peut le produire uniquement à partir de la source de carbone simple de départ. Il est aussi clair que la souche peut produire un stéroïde de la voie métabolique, par utilisation d'un substrat situé en amont dans la voie métabolique, dans la mesure où la souche de levure selon la présente invention contient tous les gènes nécessaires à la complétion de la voie métabolique de production des stéroïdes.

De préférence, la souche de levure selon l'invention produit un stéroïde ou dérivé de stéroïde qui est un dérivé du métabolisme du cholestérol, c'est à dire qui fait partie de la chaîne de métabolisme du cholestérol. Le métabolisme du cholestérol est bien connu de l'homme du métier, et est explicité dans les ouvrages de biochimie et d'endocrinologie.

Ainsi, de préférence, ledit stéroïde ou dérivé de stéroïde est compris notamment dans le groupe constitué de la 17α-hydroxy prégnénolone, le cortisol, la cortisone, la cortexolone, la 17α-hydroxy progestérone, et les dérivés de ces stéroïdes.

On peut également produire la prégnénolone et la progestérone avec une souche de levure selon la présente invention.

Ainsi, la présente invention a notamment pour objet une souche de levure génétiquement modifiée produisant, de manière autonome à partir d'une source de carbone simple, un stéroïde ou un dérivé de stéroïde, dérivé du métabolisme du cholestérol, **caractérisée en ce que** ledit stéroïde ou dérivé de stéroïde est compris dans le groupe constitué de la 17α-hydroxy prégnénolone, l'hydrocortisone, la cortexolone, la 17α-hydroxy progestérone, et les dérivés de ces stéroïdes.

Ainsi qu'il sera vu plus loin la souche de levure selon l'invention présente au moins une modification génétique choisie dans un groupe constitué de la disruption ou inactivation d'un gène endogène, la modification du promoteur d'un gène endogène, la duplication d'un gène endogène, l'introduction d'au moins un gène hétérologue, en une ou plusieurs copies, de façon épisomale ou chromosomale.

Il est d'ailleurs avantageux que la souche de levure de l'invention présente une combinaison desdites modifications géniques.

Ainsi, la présente invention a pour objet une souche de levure génétiquement modifiée produisant, de manière autonome à partir d'une source de carbone simple, un stéroïde ou un dérivé de stéroïde, dérivé du métabolisme du cholestérol, ledit stéroïde ou dérivé de stéroïde étant compris dans le groupe constitué de la prégnénolone, la 17α-hydroxy prégnénolone, le cortisol, la cortexolone, la progestérone, la 17α-hydroxy progestérone, et leurs dérivés acétylés, hydroxylés ou portant un dérivé halogéné ou un groupe méthyle, qui présente une inactivation des gènes endogènes *ATF2, GCY1* et *YPR1.*

Dans un autre mode de réalisation, la souche de levure selon l'invention présente au moins une inactivation additionnelle d'un gène endogène choisi dans le groupe constitué de *ERG5, ARE1, ARE2, ATF1* et *ADE2*.

Dans un autre mode de réalisation, la souche de levure selon l'invention présente une disruption des gènes endogènes *ERG5, ATF2, GCY1, YPR1.* Ainsi que décrit plus loin, ces gènes codent pour des protéines induisant des réactions parasites dans la levure.

Le gène *ADE2* quant à lui peut également être disrupté de façon optionnelle, notamment pour intégrer un gène hétérologue dans la souche de levure.

Dans un mode de réalisation, la souche de levure selon l'invention présente au moins un gène hétérologue intégré dans le chromosome, à au moins un locus choisi parmi *ADE2, HIS3, TRP1, LEU2, GCY1, ATF2, YPR1*, l'intégration étant effectuée de manière intragénique ou intergénique en voisinage immédiat avec l'un des loci.

Dans un mode de réalisation de l'invention, ladite souche de levure présente au moins un gène hétérologue situé sur un plasmide multicopie ou un plasmide à bas nombre de copies, ledit plasmide multicopie étant choisi parmi les plasmides à base d'un réplicon 2 micron de levure se répliquant dans *Saccharomyces cerevisiae* et ledit plasmide à bas nombre de copies étant choisi parmi les plasmides basés sur une origine de réplication ARS chromosomique avec un centromère de levure.

Pour la mise en oeuvre d'un mode de réalisation de l'invention, et ainsi que développé plus bas, la souche de levure selon l'invention présente au moins un gène ou un ADNc hétérologue choisi dans le groupe constitué du gène de la stérol Δ7-réductase et des ADNc du cytochrome P450 SCC, de l'adrénodoxine, de l'adrénodoxine réductase, du cytochrome b5, de la 3β-hydrostéroïde déshydrogénase isomérase, de la cytochrome P450 réductase, du cytochrome P450 C17, du cytochrome P450 C21, du cytochrome P450 C11, et des séquences codant pour ces protéines.

Ces gènes ou ADNc hétérologues sont sous le contrôle d'une séquence promotrice choisie dans le groupe constitué des séquences promotrices endogènes de levure *TDH3, TEF1, PGK1, CYC1, GAL10, ATF2, TIR1, ARH1, ADE2,* et du promoteur hybride *GAL10-CYC1*.

Il est nécessaire d'utiliser une séquence terminatrice pour tout gène ou ADNc hétérologue introduit, de préférence, choisie parmi les séquences terminatrices des gènes endogènes *PGK1, CYC1, ATF2, ADE2, NCP1*.

On obtient alors des blocs ou cassettes d'expression, qui consistent en un promoteur, le gène hétérologue (ou ADNc, éventuellement codant pour la protéine mature précédée par le codon ATG codant pour la méthionine (Met-mat) ou pour une protéine de fusion possédant éventuellement des signaux d'adressage vers des compartiments cellulaires), et une séquence terminatrice.

Dans un mode de réalisation, la souche de levure selon l'invention présente le bloc d'expression hétérologue stérol Δ7-réductase intégré dans le chromosome au locus ADE2.

Dans un mode de réalisation particulier de l'invention, la souche de levure comprend au moins une cassette d'expression des gènes codant pour le P450_{scc}, adrénodoxine cofacteur du P450_{scc} localisée sur un plasmide à grand nombre de copies, et elle comprend une cassette d'expression de l'adrénodoxine réductase cofacteur du P450_{scc} localisée sur un plasmide mono ou à faible nombre de copies ou intégrée dans le chromosome. De façon préférée, ces cassettes d'expression contiennent la protéine mature précédée d'une méthionine, et la protéine est localisée dans le cytosol.

Dans un mode de réalisation, la souche de levure comprend au moins une cassette d'expression choisie parmi les cassettes d'expression de la 3β-hydrostéroïde déshydrogénase isomérase, du cytochrome P450c17, du cytochrome P450c21 localisée sur un plasmide à haut nombre de copies, à faible nombre de copies ou intégrée dans le chromosome.

Dans un mode de réalisation particulier, la souche de levure selon l'invention comprend au moins une cassette d'expression pour la P45011β située sur un plasmide multicopie, la protéine produite présentant un signal d'adressage vers les mitochondries et/ou au moins une cassette d'expression pour l'adrénodoxine cofacteur de la P45011β située sur un plasmide multicopie, avec un promoteur faible (c'est-à-dire dont la force s'apparente à celle du promoteur *CYC1*), la protéine produite présentant un signal d'adressage vers les mitochondries. De façon préférée, les protéines sont produites sous forme d'un précurseur, avec un signal homologue ou hétérologue d'adressage vers les mitochondries, les protéines prenant leur forme mature dans ce compartiment cellulaire.

Il est donc intéressant de noter que dans un cas particulièrement préféré de mise en oeuvre de l'invention, on introduit dans la souche de levure deux copies du gène codant pour l'adrenodoxine, l'une d'entre elles étant destinée à exprimer la protéine dans le cytosol de la cellule, l'autre étant fabriquée de telle sorte que la protéine mature se trouve dans les mitochondries.

Dans un mode de réalisation particulier, la souche de levure comprend également au moins une cassette d'expression (promoteur d'expression tel que cité ci-dessus avec la partie codante du gène *NCP1, ATR1*, et/ou *ATR2,* avec son propre terminateur ou un terminateur tel que défini ci-dessus) située sur un plasmide multicopie, à faible nombre de copies ou intégrée dans le chromosome. Les cassettes d'expression pour *NCP1, ATR1,* et *ATR2* pourront notamment être intégrées au locus *NCP1* de *S. cerevisiae*.

Dans un mode de réalisation particulier, la souche de levure selon l'invention exprime également la protéine ARH1p, protéine homologue de l'adrénodoxine réductase des mammifères chez la levure, à un niveau supérieur au niveau d'expression physiologique. La surexpression de cette protéine peut être obtenue par des techniques bien connues de l'homme du métier, par exemple par l'apport d'une nouvelle cassette d'expression (promoteur d'expression, partie codante du gène *ARH1* avec son propre terminateur ou un terminateur tel que défini ci-dessus) en plus du gène endogène dans la levure. De manière surprenante, il a en effet été montré qu'une expression du gène ARH1 à un niveau supérieur au niveau d'expression physiologique augmente de façon significative la quantité de stéroïdes produits. Cependant cette expression ne doit pas être trop importante pour obtenir l'effet désiré. Ainsi, si une expression de la protéine ARH1 à un niveau supérieur par rapport à un niveau physiologique est souhaitable, il faut prendre garde à ne pas surexprimer trop fortement cette protéine sous peine de perdre cette augmentation de production en stéroïdes.

La souche de levure selon la présente invention peut posséder un caractère polyploïde, diploïde, haploïde ou aneuploïde, sans que cela ne nuise à la mise en oeuvre de l'invention.

Il s'agit de préférence d'une souche de *Saccharomyces cerevisiae*, notamment dérivée d'une des souches FY 1679-28c, FY 1679-18b qui sont des spores de la souche FY 1679 déposée à l'American Type Culture Collection sous le numéro 96604.

Il est utile que la souche de levure selon l'invention possède les éléments nécessaires pour l'excrétion du stéroïde produit dans le milieu de culture, afin de simplifier la purification du produit final.

L'invention concerne également un procédé de production d'un stéroïde, caractérisé en ce qu'il comprend les étapes de fermentation d'une souche de levure selon l'invention en présence d'une source de carbone simple, et de récupération du stéroïde produit.

Enfin, l'invention a également pour objet une préparation pharmaceutique comprenant une souche de levure selon l'invention, avec éventuellement un excipient pharmaceutiquement acceptable, un tel excipient étant bien connu de l'homme du métier.

Bien que la souche de levure selon l'invention produise un stéroïde de façon autonome à partir d'une source de carbone simple, on peut également lui fournir du cholestérol ou une structure apparentée, ou un substrat déjà présent en tant que dérivé du cholestérol afin d'obtenir les produits situés en aval. La possibilité de pouvoir entrer à n'importe quelle étape, notamment au niveau prégnénolone ou postérieur de la voie de métabolisme du stéroïde recherché permet donc notamment de pouvoir fournir à la levure des substrats non naturels, qui mènent à la synthèse de stéroïdes non naturels et substitués, notamment fluorés.

Dans un premier mode de réalisation, la souche de levure selon la présente invention permet notamment de produire le stéroïde recherché (notamment le cortisol), en quantité supérieure à 10 mg/l, de préférence supérieure à 50 mg/l, de façon plus préférée 80 mg/l de façon plus préférée 100 mg/l, de façon la plus préférée 200 mg/l.

Dans un autre mode de réalisation, le stéroïde d'intérêt (de façon préférée l'hydrocortisone) est présent dans une proportion supérieure à 20 %, de préférence 25 %, de façon plus préférée 30 %, de façon plus préférée 35%, de façon plus préférée 40 %, de façon plus préférée 50 %, de la façon la plus préférée 65% du total des stéroïdes produits par la souche selon l'invention (notamment les intermédiaires de synthèse).

Afin que la souche de levure selon la présente invention puisse produire les stéroïdes d'intérêt, il est nécessaire qu'elle présente des modifications génétiques. Ainsi, la souche de levure selon l'invention présente au moins une modification génétique choisie dans groupe constitué de la disruption ou inactivation d'un gène endogène, la modification du promoteur d'un gène endogène, la duplication d'un gène endogène, l'introduction d'au moins un gène hétérologue (notamment un bloc d'expression avec promoteur et/ou terminateur homologue et une partie codante hétérologue), en une ou plusieurs copies, de façon épisomale ou chromosomale.

De façon préférée, la souche de levure selon l'invention présente plusieurs (au moins quatre) modifications génétiques telles qu'énoncées ci-dessus.

Ainsi, certains gènes endogènes de la levure sont favorablement inactivés ou disruptés. On peut inactiver ou disrupter les gènes par l'introduction, dans la séquence codante, d'un gène exogène (notamment un bloc d'expression avec promoteur et/ou terminateur homologue et une partie codante hétérologue) tel que décrit plus bas, et/ou d'un marqueur de sélection. On peut également modifier les promoteurs de ces gènes afin de réduire le niveau d'expression.

Le gène de levure *ATF2* (Cauet *et al*.,1999) code pour une acétyl transférase qui utilise la prégnénolone comme substrat et sa disruption permet de supprimer cette réaction parasite d'acétylation, dont le produit ne peut alors être utilisé, et d'augmenter ainsi le rendement en stéroïde d'intérêt. Ainsi, les rendements peuvent être multipliés par des valeurs comprises entre 3 et 7 après inactivation du gène *ATF2.*

Les gènes *GCY1* et *YPR1* codent pour des aldo-kéto-réductases. Ces gènes sont avantageusement inactivés ou disruptés. Ces deux gènes font partie d'une famille de 6 gènes plus ou moins homologues et supposés coder tous les six pour des aldo-kéto-réductases. Toutefois, les produits de ces deux gènes sont les plus actifs sur les substrats ici envisagés, notamment *GCY1*, et leur inactivation est donc extrêmement intéressante pour l'obtention de l'hydrocortisone.

Ainsi que précisé ci-dessus, il est intéressant d'inactiver le gène *ERG5*, afin d'accumuler un substrat qui présente une structure la plus proche possible de la structure du cholestérol. Toutefois, il a été montré que la levure selon l'invention peut malgré tout produire les stéroïdes d'intérêt malgré l'activité de ce gène. Toutefois, afin d'optimiser les rendements, il peut être utile de l'inactiver par mutation, délétion et/ou insertion.

On peut aussi, sans que ceci ne soit réellement critique pour la réussite globale de la production de stéroïdes avec la levure selon la présente invention, inactiver d'autres gènes, tels *ARE1, ARE2, ADE2* ou *ATF1.* Ces gènes codent tous pour des protéines dont l'absence peut améliorer le rendement global de synthèse du stéroïde d'intérêt.

Ainsi que décrit dans l'article de Duport *et al*., précédemment cité (Duport *et al*., 1998), il est indiqué que la présence d'un bloc d'expression avec promoteur et/ou terminateur homologue et une séquence codant pour la Δ7-réductase est utile en ce qu'elle permet d'effectuer la désaturation de la double liaison 7-8 de l'ergostérol et de ses dérivés, et d'obtenir ainsi un ou plusieurs précurseurs de structure plus proche de la structure du cholestérol, substrat de départ pour la production de prégnénolone. Ainsi, il est avantageux que la souche de levure selon la présente invention contienne ce bloc d'expression. Dans un cas particulier, ledit bloc d'expression de la Δ7-réductase est intégré dans le génome de la levure, de façon préférée au locus du gène *ADE2,* menant par là même à la disruption du gène *ADE2.* Le promoteur utilisé pour la transcription est un promoteur inductible tel que *GAL10-CYC1,* ou un promoteur constitutif, tel le promoteur *GAL10-GAL10-CYC1* qui est dérégulé dans la souche CA10 décrite dans Duport *et al*., 1998. La protéine utilisée est préférentiellement issue d'*Arabidopsis thaliana* (mais peut aussi être issue d'une espèce de mammifère), l'ADNc étant cloné sous la forme native (ADN complémentaire juste après une méthionine d'initiation de traduction), et sous le contrôle d'un terminateur de transcription classique chez la levure tel *PGK1.*

Il est à noter que l'activité du gène Δ7-réductase dans la levure a été décrite par Lecain *et al*., 1996, dont le contenu technique (notamment les séquences du gène Δ7-réductase et les constructions et méthodes opératoires) est incorporé par référence dans la présente demande.

La première étape, est la production de prégnénolone, obtenue après introduction, dans la levure, des enzymes permettant de transformer normalement le cholestérol en prégnénolone. Dans le présent cas, il s'agit de l'enzyme permettant le clivage de la chaîne latérale (P450_{scc} pour side chain cleavage), avec deux coenzymes (adrénodoxine, *ADX*, et adrénodoxine réductase, *ADR*). La transformation de la levure avec ces blocs d'expression respectifs est décrite dans Duport *et al*., 1998, précédemment cité.

On utilise de préférence un ADN complémentaire codant pour les protéines matures, avec une méthionine ajoutée à l'extrémité N-terminale pour permettre la traduction. On utilise des promoteurs tels que le promoteur hybride *GAL10-CYC1,* ou le promoteur *TEF1,* pour assurer la transcription des ADNc. On utilise les terminateurs classiques, notamment le terminateur *PGK1.*

Les différents ADNc codant pour les protéines P450_{scc}, ADR ou ADX peuvent être d'origine d'un vertébré, par exemple humaine ou bovine, mais aussi rat ou poisson. Les gènes codant pour ces protéines sont de préférence placés sur des plasmides, on préfère pour P450_{scc} et ADX un plasmide multicopie à faible ou grand nombre de copies, notamment dérivé d'un plasmide 2 micron de levure, alors qu'on utilise plutôt un plasmide monocopie ou à faible nombre de copies pour l'expression de ADR. Le bloc d'expression pour *ADR* peut aussi être intégré dans le chromosome de la levure. Ceci permet de contrôler l'expression de l'ADR, car il semble que trop d'expression nuise à l'activité scc recherchée.

Les protéines sont de préférence exprimées pour pouvoir exercer leur activité dans le cytosol.

L'étape suivante est la conversion de la prégnénolone en 17α-hydroxy progestérone, par l'action conjuguée de la 17α-hydoxylase (P450c17) et de la 3β-OH-stéroïde déshydrogénase (3β-HSD).

Pour l'expression de ces deux protéines, on utilise de préférence des promoteurs forts, tels *TEF1, TDH3, GAL10-CYC1.* Toutefois, un promoteur plus faible tel *CYC1* peut également convenir. Les terminateurs utilisés sont classiques, notamment provenant des gènes *PGK1* ou *NCP1.* On exprime les ADN complémentaires codants pour les protéines complètes. L'espèce d'origine de ces protéines ne semble pas modifier les résultats obtenus, ainsi on peut utiliser des protéines d'origine humaine (notamment l'un ou l'autre des deux isotypes de la 3β-HSD), bovine, ou d'autres organismes (notamment de poissons). Il s'agit, pour ces deux protéines, d'obtenir la meilleure expression possible, et on peut donc les exprimer sur des plasmides mono ou multicopies à faible ou grand nombre de copies, ou en ayant intégré les blocs d'expression dans au moins un chromosome de la levure.

On recherche ensuite la conversion de la 17α-hydroxy progestérone en désoxycortisol, par l'intermédiaire de la P450c21, qui permet une hydroxylation en 21. Il s'agit d'exprimer la protéine, à partir de son ADNc, de la meilleure façon possible. Pour ce faire, on utilise un promoteur fort (*TEF1, TDH3, GAL10-CYC1...*)*,* voire le promoteur *CYC1,* et un terminateur classique (notamment *PGK1*) pour concevoir l'unité transcriptionnelle. Celle-ci est placée sur un plasmide mono ou multicopies, à faible ou grand nombre de copies, ou bien intégrée dans le génome de la levure. Il est à noter que l'espèce d'origine semble ici importante, et qu'il est préférable d'utiliser la P450c21 d'origine humaine.

Le désoxycortisol est ensuite converti en cortisol, sous l'action du système P450c11, qui contient la P450c11, permettant l'hydroxylation en 11-β, une adrénodoxine et une adrénodoxine réductase, en tant que cofacteurs.

Les inventeurs de la présente demande ont montré que les résultats obtenus étaient meilleurs lorsque ce dernier système est exprimé dans la membrane interne des mitochondries des levures. Ainsi, il est intéressant de produire des protéines de fusion, qui portent en tant que précurseur la séquence d'adressage mitochondrial du précurseur de la protéine Cox6p de la levure.

On utilise de préférence également l'ADNc codant pour les protéines matures. La protéine P450c11 est ainsi la protéine d'origine humaine, bovine ou une protéine hybride humaine-bovine. Cette dernière construction est la forme préférée pour la mise en oeuvre de l'invention. Le gène utilisé dans l'unité transcriptionnelle est de préférence sous le contrôle du promoteur *CYC1*. Il est intéressant de positionner un intron de β-globine de lapin et un terminateur du gène de l'hormone de croissance humaine en 3' de la séquence codante. L'unité transcriptionnelle est placée de préférence sur un plasmide multicopie.

L'adrénodoxine est utilisée sous sa forme mature, placée avec une séquence d'adressage vers les mitochondries, par exemple choisie parmi celles des précurseurs des protéines Cox6p, Cox4p, fumarase, ARH1p, F9 ATPase, et sous le contrôle d'un promoteur choisi notamment parmi *TDH3, TEF1*, *CYC1*. On préfère une protéine d'origine bovine ou humaine. Il convient de placer un terminateur dans l'unité transcriptionnelle, qui peut être *PGK1*. On exprime le bloc d'expression de préférence à partir d'un plasmide multicopie.

La protéine faisant fonction d'adrénodoxine réductase est la protéine ARH1p, endogène de la levure, qui est normalement exprimée dans les mitochondries de l'hôte. De façon préférée, on transforme toutefois la levure de telle façon que la souche hôte contienne une copie naturelle du gène *ARH1* et une seconde copie du gène *ARH1* sous le contrôle du promoteur *CYC1*. L'activité de cette protéine est indispensable pour obtenir l'effet recherché, et il a même été observé que l'inactivation du gène est létale pour la levure (Lacour *et al*., 1998). Il convient de noter que la surexpression de ce gène semble toxique pour l'organisme, et que le promoteur choisi doit donc permettre un niveau d'expression qui mène à l'activité 11-β hydroxylase recherchée sans être délétère pour la levure. De manière surprenante, il a en effet été montré qu'une expression du gène ARH1 à un niveau supérieur au niveau d'expression physiologique augmente de façon significative la quantité de stéroïdes produits. Cependant comme il a été dit ci-dessus, cette expression ne doit pas être trop importante pour obtenir l'effet désiré. Ainsi, si une expression de la protéine ARH1 à un niveau supérieur par rapport à un niveau physiologique est souhaitable, il faut prendre garde à ne pas surexprimer trop fortement cette protéine sous peine de perdre cette augmentation de production en stéroïdes. A titre d'exemple, une intégration de la cassette d'expression pour *ARF1*, comportant comme promoteur le promoteur CYC1, au niveau du locus *LEU2* de *S*. *cerevisiae* donne des niveaux d'expression et des résultats satisfaisants. A l'inverse l'intégration au même locus d'une cassette comportant le promoteur *TEF1*, reconnu comme beaucoup plus fort que le promoteur CYC1, donne des résultats moins intéressants.

Ainsi, on introduit de préférence deux copies d'une unité transcriptionnelle codant pour la protéine co-enzyme ADX, l'une d'entre elles ayant une activité à l'extérieur des mitochondries et notamment dans le cytosol, l'autre ayant une activité dans les mitochondries de la cellule hôte.

Il est également possible d'introduire d'autres gènes, notamment les gènes codant pour des protéines ayant une activité NADPH P450 réductase, tels *NCP1* (réductase de levure aussi appelé *CPR1*)*, ATR1* ou *ATR2* (réductases de plantes), réductase humaine. Ces protéines améliorent les activités P450c17 et P450c21. On utilise soit le promoteur endogène (*NCP1*), ou on met les ADN codant pour les protéines sous le contrôle de promoteurs tels *GAL10-CYC1, CYC1, TEF1...*

Il est également possible d'introduire le gène *TGL1*, codant pour une protéine ayant une activité de désestérification, sous le contrôle d'un promoteur fort tel *GAL10-CYC1,* sur un plasmide multicopie ou monocopie. Ceci permet de réduire l'effet des réactions d'estérification parasites des stérols pouvant subsister même après l'inactivation de *ATF2,* et notamment celles produites par le produit des gènes *ARE1* et *ARE2*.

On peut aussi ajouter un plasmide exprimant le cytochrome b5 de levure ou une autre espèce, qui est un cofacteur dans plusieurs des réactions définies ci-dessus.

On peut aussi, lorsque l'on désire produire de la DHEA, introduire un plasmide codant pour la desmolase (P450 17α), à faible ou grand nombre de copies, sous le contrôle d'un promoteur tel *GAL10-CYC1, CYC1, TEF1*, avec un terminateur *PGK1.*

On peut également introduire un ADNc codant pour le cytochrome P450c17 ayant une activité lyase, par exemple celui d'origine humaine, en présence d'un excès de NADPH P-450 réductase, comme les réductases de mammifère, *NCP1*, *ATR1*, ou *ATR2.* Ces unités transcriptionnelles utilisent de préférence un promoteur fort.

Enfin, il est possible de restaurer l'activité des gènes endogènes *ERG6* et *ERG2,* inhibés par la prégnénolone, en les plaçant sous le contrôle d'un promoteur constitutif fort.

On peut aussi introduire d'autres gènes hétérologues, codant notamment pour une protéine à activité 24,25 stérol réductase, ou le gène *HMG1*, présent dans la voie de synthèse du cholestérol chez l'homme. Il est également intéressant d'introduire le gène *MDR1* humain, qui code pour une pompe non inhibée par l'accumulation des stérols anormaux qui apparaissent en raison de l'inhibition du gène *ERG6* par la prégnénolone. Ceci permet d'expulser ces produits dont une trop grande accumulation pourrait s'avérer toxique pour la cellule hôte.

On peut aussi sur-exprimer le gène *PDR12* de levure qui aura un effet de détoxification pour les stéroïdes pouvant inhiber la croissance de la levure.

Il est entendu que lorsque l'on parle de gène ci-dessus, on entend non seulement le fragment d'ADN codant pour la protéine présentant l'activité recherchée (et notamment le fragment d'ADNc représentant en particulier les formes matures), mais aussi les promoteurs (notamment *TEF1, GAL10-CYC1, CYC1, TDH3*) et les terminateurs (en particulier *PGK1*). Ces unités transcriptionnelles sont introduites préférentiellement sur des plasmides à faible ou grand nombre de copies, ou intégrés dans le chromosome de la levure.

Il est également entendu que, suivant le but recherché, et notamment le stéroïde que l'on désire produire, il est possible de n'introduire que certains des gènes codant pour les différentes protéines à chaque étape, et de fournir un intermédiaire à la levure afin d'obtenir un stéroïde en aval dans la chaîne de métabolisme.

Il est aussi facile de ne pas introduire les gènes permettant d'obtenir les produits situés très en aval, et de pouvoir donc s'arrêter relativement haut dans la chaîne du métabolisme.

La levure utilisée pour la mise en oeuvre de la présente invention sont de préférence :
- la souche FY1679-28c, décrite dans Duport *et al*., 1998, qui possède le génotype (*MAT*α, rho⁻, *ura3*-52,*trp1Δ63, leu2Δ1, his3Δ200*, *GAL2, fen1*). Cette souche a également été décrite par Thierry *et al*., 1990.
- la souche FY1679-18b, possédant le génotype (*MATa*, rho⁻, *ura3-52, trp1Δ63, leu2Δ1*, *his3Δ200*, *GAL2,fen1*).

Ces deux souches possèdent donc un génotype identique, et un signe opposé.

### DESCRIPTION DES FIGURES

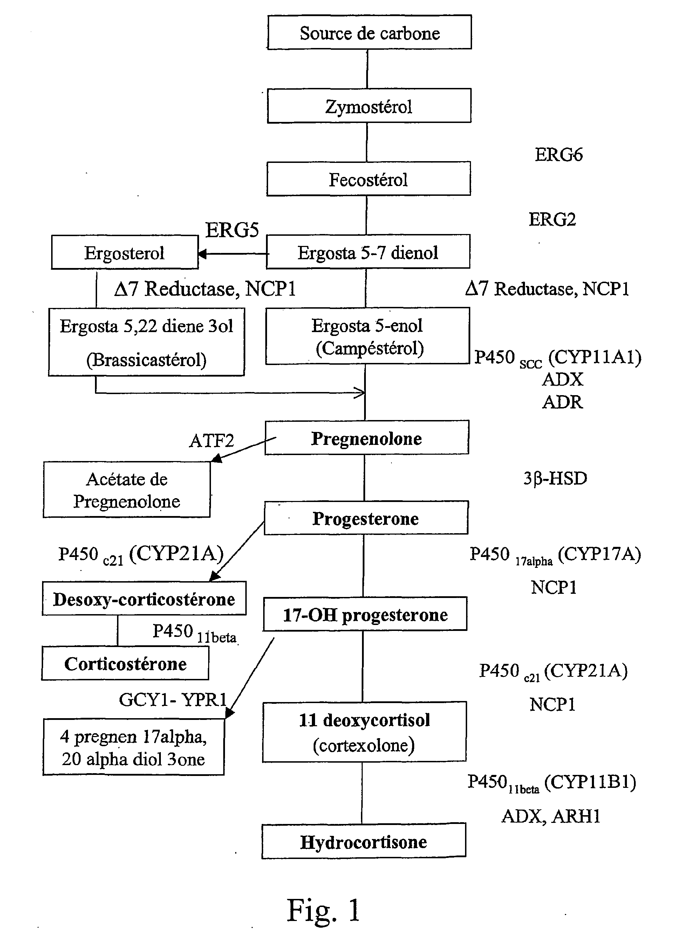

Figure 1 : Représentation schématique d'une voie de biosynthèse de l'hydrocortisone telle que l'on peut l'obtenir selon l'invention.

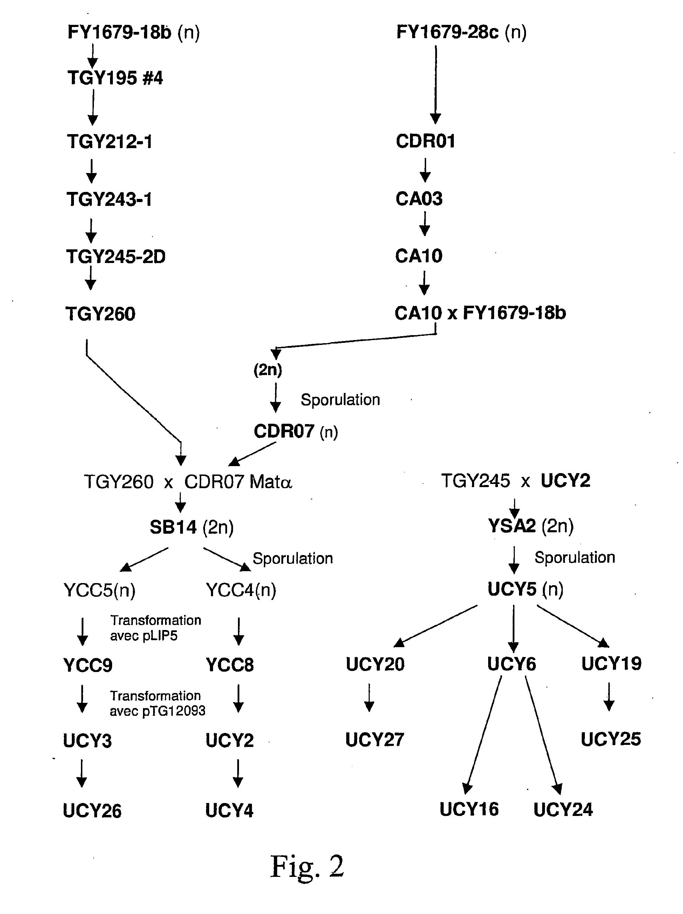

Figure 2 : Représentation schématique de construction de souches de levures exemplifiées selon l'invention.

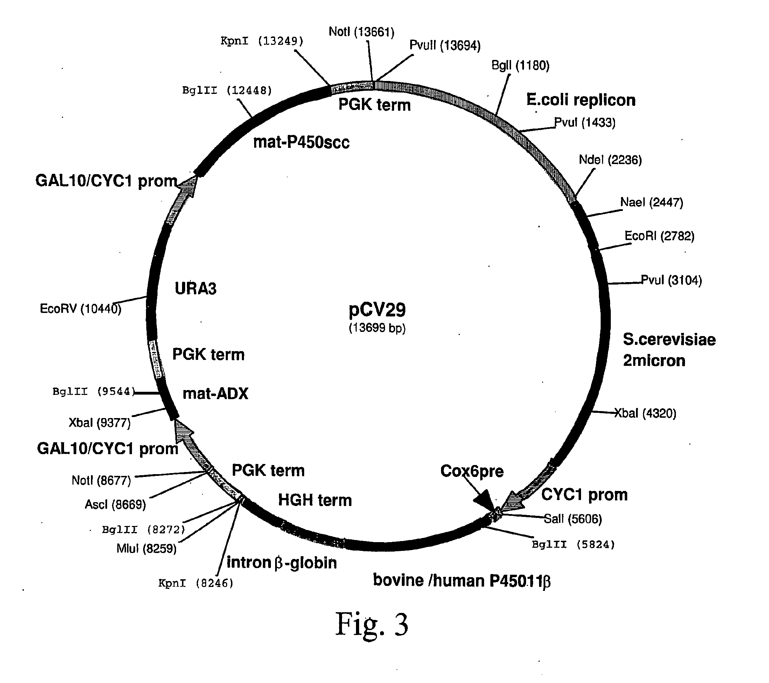

Figure 3 : Carte du plasmide pCV29. PGK term : terminateur *PGK*. GAL10/CYC1 prom : promoteur *GAL10*/*CYC1*. HGH term : terminateur du gène de l'hormone de croissance humaine. Intron β-globin: intron du gène de la β-globine de lapin. CYC1 prom : promoteur *CYC1*. PGK term : terminateur *PGK.* S. cerevisiae 2 micron : origine de replication 2 micron de *S. cerevisiae.* Cox6pre : préséquence de la cytochrome oxidase sous unité 6. Bovine/human P450 11β : ADNc fusionné bovin/humain de la P45011β. mat-ADX : forme mature de l'ADX avec une méthionine en NH2 terminal. E.coli replicon : Replicon *E. coli*

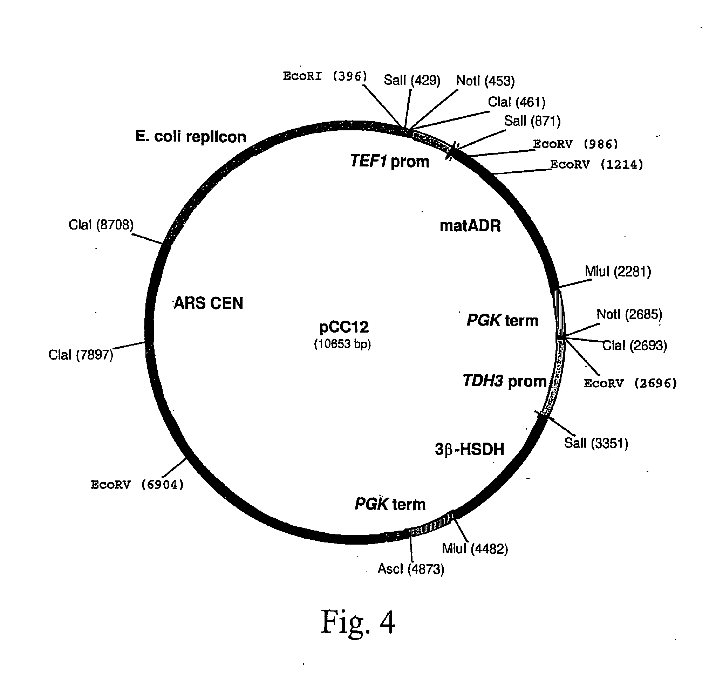

Figure 4 : Carte du plasmide pCC12. CYC1 prom : promoteur *CYC1.* PGK term : terminateur *PGK.* ARS CEN : origine de replication chromosomique de *S. cerevisiae.* E.coli replicon : Replicon *E. coli.* TDH3 prom : promoteur *TDH3.* 3β-HSDH : ADNc de la 3β hydroxy stéroide déhydrogénase. matADR : forme mature de l'ADR précédée d'une méthionine.

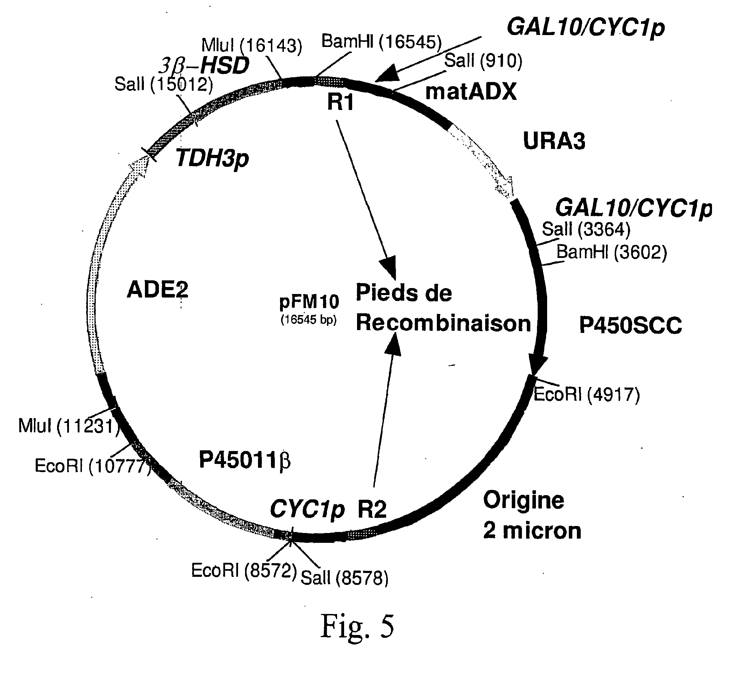

Figure 5 : Carte du plasmide pFM10. *CYC1p* : promoteur *CYC1*. P45011β : ADNc fusionné bovin/humain de la P45011β. ADE2 : gène *ADE2* de la levure. *TDH3p :* promoteur *TDH3*. *3*β-*HSD* : ADNc de la 3β hydroxy stéroide déhydrogénase. R1 : pied de recombinaison dont la séquence est donnée en SEQ ID N° 39. *GAL10*/*CYC1p :* promoteur *GAL10*/*CYC1.* matADX : ADNc codant pour la forme mature de l'ADX. URA3 : gène *URA3* de la levure. P450_{scc} : ADNc codant pour la forme mature du P450_{scc} (CYP11A1). Origine 2 micron : origine de replication 2 micron de *S. cerevisiae.* R2 : pied de recombinaison dont la séquence est donnée en SEQ ID N°40.

### EXEMPLES

Les exemples ci-dessous décrivent un mode de mise en oeuvre de la présente invention et ne doivent pas être considérés comme limitant l'invention.

On part, pour les constructions des souches de levure FY1679-28c et FY1679-18b décrites ci-dessus.

### Exemple 1 : Disruption du gène YPR1

La construction de l'interruption du gène *YPR1* (YDR368w) par le gène *URA3* dans le plasmide pPOLYIII a été obtenu par 4 PCR successives. D'une part trois PCR indépendantes ont été réalisés pour obtenir la partie 5' du gène *YPR1* (PCR 5), le gène *URA3* fonctionnel bordé par des séquences *YPR1* (PCR 6), la partie 3' du gène *YPR1* (PCR 7).

L'ADN de la PCR5 a été obtenu par amplification sur une matrice d'ADN génomique avec les oligonucléotides OTG11314 (SEQ ID N° 1) et OTG11315 (SEQ ID N° 2) de même l'ADN de la PCR7 est obtenu par amplification à l'aide des oligonucléotides OTG11316 (SEQ ID N° 3) et OTG11317 (SEQ ID N° 4) sur la même matrice.

Le gène *URA3* flanqué de région *YPR1* 5' et 3' est amplifié à l'aide des oligonucléotides OTG11463 (SEQ ID N° 5) et OTG11464 (SEQ ID N° 6) sur une matrice pTG10054 décrit dans Degryse *et al*., 1995 incorporé ici par référence en ce qui concerne la description de ce plasmide.

Les produits des PCR5, PCR6 et PCR7 ont été mélangés de façon équimoléculaire puis amplifiés par PCR à l'aide des oligonucléotides OTG11314 (SEQ ID N° 1) et OTG11317 (SEQ ID N° 4) pour obtenir un produit de PCR8.

Ce produit PCR8 a été digéré par l'enzyme *Xho*I puis sous cloné dans le plasmide pPOLYIII (décrit par Lathe *et al*., 1987 et incorporé ici par référence en ce qui concerne la description de ce plasmide) digéré par *Xho*I pour donner le plasmide pTG12011. L'orientation de l'insertion dans le plasmide pPOLYIII a été déterminée par digestion par les enzymes *NcoI* et *EcoRI*.

Le plasmide pTG12011 qui permet la disruption du gène parasite *YPR1* par le gène *URA3* est digéré par l'enzyme *Xho*I*.* Le produit de la digestion est utilisé pour transformer la souche FY1679-18b en utilisant la méthode au chlorure de lithium bien connue de l'homme du métier. Les transformants sont sélectionnés sur un milieu dépourvu en uracil. Les transformants sont analysés par amplification par PCR en utilisant les oligonucléotides qui ont servi à la construction du plasmide pTG12011.

Les clones positifs dans ce test sont ensuite criblés par la méthode de bioconversion du 170H progestérone décrite plus bas en présence de glucose comme source de carbone. La capacité de bioconversion est analysée en HPLC comme décrit par Dumas *et al*., 1996 et Degryse *et al*., 1999 dont les contenus sont incorporés par référence à la présente demande, notamment les explications des études de bioconversion ou comme décrit dans Kuronen *et al*., 1999. Un clone TGY195#4 est sélectionné pour de nouvelles caractérisations. La souche TGY195#4 est transformée en utilisant à la fois le plasmide YRp7 (Parent *et al*., 1985, qui est incorporé par référence pour ce qui est de la description de ce plasmide) (1µg) et 5µg de plasmide pTG12045 (décrit plus bas) digéré par *Not*I*.* Les souches transformées sont sélectionnées sur un milieu dépourvu en tryptophane. Des colonies (678 colonies) sont repiquées sur un milieu contenant du tryptophane (pour se débarrasser du plasmide YRp7) et sur un milieu contenant du tryptophane et du 5 fluoro orotate (5F0) pour sélectionner les colonies qui ont perdu le gène *URA3* interrompant le gène *YPR1.*

### Exemple 2 : Construction de différents plasmides.

Pour la surexpression de la protéine P450c21 dans la levure deux types de promoteur ont été utilisés, *TEF1* (« Transcription elongation factor 1 ») et *TDH3* (« glyceraldehyde-3-phosphate dehydrogenase 3). Dans tous les cas, le terminateur de transcription est le terminateur PGK. Dans ces plasmides, le fragment *Sal*I, *Mlu*I porte l'ADMIc de la P450c21 humaine.

### a) Construction des plasmides pTG10470 et pTG10469

Le plasmide pTG10289 a été obtenu par modification du pMAc21 (Wu *et al*, 1991) par digestion avec *Kpn*I*, Mlu*I et introduction de l'oligonucléotide OTG5868 (SEQ ID N° 27).

L'ADNc de ce plasmide provient de l'American Type Culture Collection (ATCC, Rockville, Maryland, USA) sous le nom de pc21/3c: C'est le fragment *EcoR*I*-BamH*I de 1,6Kb qui a servi de base pour la construction des différents plasmides. Les modifications apportées sont décrites dans l'article ci-dessus et dans l'article de Hu *et al*, 1990.

Dans cette manoeuvre, la partie non codante de la P450c21 du plasmide pMAc21 qui contient la cassette d'expression de la P450c21 a été éliminée ainsi que le site *Kpn*I qui s'y trouvait.

Le plasmide pTG10292 a été obtenu par transfert de l'ADNc c21 humain (fragment *Sal*I*, Mlu*I) du plasmide pTG10289 dans le plasmide pTG10031 (décrit dans Degryse *et al*., 1995, qui est incorporé par référence à la demande) à l'aide des sites *Sal*I et *Mlu*I*.*

Le plasmide pTG10475 a été obtenu par PCR et recombinaison. En effet à partir du plasmide pTG10292, un fragment de l'ADNc P450c21 humain représentant approximativement 250 nucléotides a été amplifié à l'aide des oligonucléotides OTG7410 (SEQ ID N° 7) et OTG5927 (SEQ ID N° 8). Ce fragment représente la séquence codante, de la P450c21 humaine d'un site *Sal*I et de la séquence AAAA.

Ce fragment a été digéré par *Sal*I puis ligué sur le fragment linéaire de pTG10292 digéré par *Sal*I et ensuite une expérience de recombinaison a été réalisée dans la souche BJ5183 décrite par Degryse *et al*., 1995.

Le plasmide obtenu pTG10475 porte un ADNc de la P450c21 avec une séquence codante identique à celle de l'ADNc naturel, contrairement au plasmide pMAc21, sur un fragment compatible avec les vecteurs utilisés généralement par les inventeurs, c'est à dire un fragment bordé par les sites de restriction *Sal*I et *Mlu*I*.* Ce fragment possède l'environnement suivant autour du codon ATG d'initiation de la traduction GTCGACAAAAATGCTGCTCCTGGGCCTGCTGC (SEQ ID N° 9).

A partir de ce plasmide le fragment *Sal*I*, Mlu*I portant l'ADNc de la P450c21 humaine a été transféré dans le plasmide pTG10158 (Degryse *et al* 1995) par clonage classique pour donner le plasmide pTG10472.

Ce même fragment *Sal*I *Mlu*I du plasmide pTG10472 a été ensuite transféré par clonage classique dans le plasmide pTG10085 (Degryse *et al* 1995) pour donner le plasmide pTG10469. Ce plasmide possède donc l'ADNc de la P430c21 humaine sous le contrôle du promoteur *TEF1*, avec le terminateur *PGK1*.

Ce même fragment portant l'ADNc P450c21 sur un fragment de restriction *Sal*I et *Mlu*I est transféré dans le plasmide pTG10092 par recombinaison dans la souche BJ5183 pour donner le plasmide pTG10470 (Degryse *et al*., 1996).

Le plasmide pTG10470 porte donc l'ADNc de la P450c21 humaine sous contrôle du promoteur *TEF1* et d'un terminateur *PGK*1 avec un marqueur de sélection *URA3-d* avec l'environnement du codon initiateur ATG décrit précédemment.

### b) Construction du plasmide pTG12036.

Le plasmide pTG12036 a été construit en 4 étapes à partir du pTG10802. Le plasmide pTG10801 (qui est à l'origine du plasmide pTG10802) est un plasmide de type pUC dans lequel une suite de sites de restriction a été insérée entre les sites *Xho*I et *Xho*I*.* Cette suite de sites comprend les sites *Hind*III, *Snab*I, *Cla*I et *Spe*I*.*

Entre les sites *Hind*IIT et *Cla*I*,* la cassette *Hind*III *Cla*I du'pTG10470 comprenant le promoteur *TEF1*, l'ADNc P450c21 humain et le terminateur *PGK1* a été inséré entre les sites *Hind*III et *Cla*I de pTG10801 pour donner pTG10802.

Ce plasmide a été ensuite digéré par *Xho*I et la cassette introduite est donc délétée pour introduire un fragment de PCR bordé par des sites *Xho*I*.* Ce fragment de 2,5Kb provient de l'amplification par la paire d'oligonucléotides OTG11844 (SEQ ID N° 10) et OTG11845 (SEQ ID N° 11) sur le plasmide pTG12010#40 (cf. ci dessous) pour obtenir un fragment bordé par des *Xho*I contenant le gène *GCY1* interrompu par le gène *URA3* bordé en 5' par un site de restriction *Cla*I*.*

Ce fragment a été cloné entre les sites *Xho*I du plasmide pTG10802 pour obtenir le plasmide pTG12035. Dans le but d'introduire le site *Hind*III manquant, le plasmide pTG12010#36 a été utilisé. Ce plasmide est essentiellement identique au pTG12010#40 mais possède un site *Hind*III en 3' du gène *URA3* à la limite avec le gène *GCY1* mais ne possède pas de site *Cla*I en 5' du gène *URA3* à la jonction avec le gène *GCY1* (cf.ci-dessous). Par recombinaison *in vivo* dans *E. coli*, entre le fragment *Nco*I *BamH*I de 2,2Kb qui porte de 5' vers 3' un fragment du gène *URA3,* le fragment 3' du gène *GCY1* et enfin une partie du plasmide pTG12035 c'est à dire le grand fragment *Stu*I*, Afl*II de 4,45Kb. On obtient le plasmide pTG12036.

Le plasmide obtenu pTG12036 possède le gène *GCY1* interrompu par le gène *URA3* bordé par des sites *Cla*I et *Hind*III respectivement en 5' et 3'. Ce fragment est ensuite remplacé par la cassette d'expression de la P450c21 porté par le fragment 2,33Kb *Cla*I*, Hind*III du plasmide pTG10469 (voir plus haut) pour obtenir le plasmide pTG12086.

### c) Construction du plasmide pTG12045.

Le site unique *Sph*I du plasmide pPOLYIII est détruit par insertion de la paire d'oligonucléotides complémentaires OTG11975 (SEQ ID N° 12) et OTG11976 (SEQ ID N° 13).

Le site *Sph*I du pPOLYIII est détruit et remplacé par un site *Cla*I pour donner le plasmide pTG12040. Dans le plasmide pTG12040 entre les sites uniques *Cla*I et *EcoR*I*,* on introduit un fragment d'ADN génomique *Cla*I *EcoR*I correspondant à la partie 3' de 0,7Kb du gène *YPR1* obtenu par amplification avec les oligonucléotides OTG11981 (SEQ ID N° 14) et OTG11982 (SEQ ID N° 15) pour donner le plasmide pTG12041.

Dans ce plasmide pTG12041 de 2,84Kb, la partie 5' du gène *YPR1* (0.66Kb) amplifiée par les oligonucléotides OTG11314 (SEQ ID N° 1) et OTG11980 (SEQ ID N° 16) à partir d'ADN génomique de levure sauvage est clonée sous forme d'un fragment *Xho*I *Hind*III entre les sites *Sal*I et *Hind*III du plasmide ATG12041.

On obtient le plasmide pTG12042 de 3,5Kb. Ce plasmide porte le gène *YPR1* interrompu par les sites *Cla*I et *Hind*III. Entre ces sites la cassette du cytochrome P450c21 est clonée sous forme d'un fragment *Cla*I *Hind*III de 2,33 Kb provenant du plasmide pTG10469. On obtient ainsi le plasmide pTG12045.

### d) Construction des plasmides pTG12010#36 et #40

Le plasmide pTG 12010 a été construit sur une base du plasmide pUC19 (Yanisch-Perron et al., 1985) tandis que le plasmide pTG12011 a été construit sur une base du plasmide pPOLYIII (Lathe *et al*., 1987).

La construction de la disruption du gène *GCY1* par le gène *URA3* dans le plasmide pUC19 a été obtenue par quatre amplifications par PCR successives. D'une part trois PCR indépendantes ont été réalisés pour obtenir la partie 5' du gène *GCY1* (PCR1), le gène *URA3* fonctionnel bordé par des séquences *GCY1* (PCR2), la partie 3' du gène *GCY1*(PCR3)*.*

Les parties 5' et 3' du gène *GCY1* à l'aide des couples OTG11285, OTG11286 et OTG11287, OTG11289 (respectivement SEQ ID N° 17 à SEQ ID N° 20) sur une matrice d'ADN génomique de la souche FY1679-28c,

Le gène *URA3* flanqué de séquences *GCY1* (de façon à obtenir une délétion d'une partie de la séquence codante du gène *GCY1*) est amplifié à l'aide des oligonucléotides OTG11305 (SEQ ID N° 21) et OTG11306 (SEQ ID N° 22) à partir d'une matrice plasmide pTG10054 linéarisé (Degryse *et al*., 1995).

Les conditions de tampon et de concentration en matrice et amorces pour l'amplification sont décrites par le producteur ou fabricant de l'enzyme *Taq* ADN polymerase, et en particulier pour l'enzyme elongase développé par Life Technologies. Les cycles de températures sont les suivants: un premier cycle de 6'30 pour dénaturer amorce et matrice puis 30 cycles de 30s à 93°C, 2 mn à 54°C et 3mn à 68°C, le dernier cycle est de 5 mn à 72°C. Les produits PCR1, PCR2 et PCR3 sont mélangés de façon équimoléculaire et amplifiés de nouveau à l'aide des oligonucléotides OTG 11285 (SEQ ID N° 17) et OTG11289 (SEQ ID N° 20). Le produit final PCR4 d'une taille de 2,9 Kb est ensuite sous cloné entre les sites de restriction *Kpn*I et *BamH*I du plasmide pUC19 pour obtenir le plasmide pTG12010.

La structure du plasmide a été vérifiée par profil de restriction et séquençage nucléotidique des extrémités.

Le clonage du pTG12010 a permis en fait d'obtenir deux versions de ce plasmide, la version pTG12010#40 (pTG12040 clone 40) et ATG12010#36. La volonté initiale était d'obtenir le gène *GCY1* interrompu par le gène *URA3* bordé par les sites de *Cla*I et *Hind*III respectivement en 5' et en 3' du gène. En fait deux plasmides différents ont été obtenus, qui différent uniquement par la présence ou l'absence de sites *Cla*I et *Hind*III aux extrémités du gène *URA3.* Le plasmide pTG12010#40 possède un site de restriction *Hind*III à l'extrémité 3'du gène *URA3* mais pas de site *Cla*I en 5'. Le plasmide pTG12010 #36 ne possède pas de site *Hind*III à l'extrémité 3' mais un site *Cla*I à l'extrémité 5' du gène. Cette propriété est utilisée pour obtenir le plasmide qui possède le gène *URA3* bordé par les sites *Hind*III et *Cla*I interrompant la séquence codante de *GCY1*.

### e) Construction du plasmide pTG12086

Ce plasmide sert à l'intégration d'une cassette d'expression pour la P450c21 ainsi qu'à la disruption du gène *GCY1* dans le même temps. Ce plasmide a été construit à partir du plasmide pTG12036 et du plasmide pTG10614.

La construction du plasmide pTG10614 a été réalisée comme suit. Ce plasmide a été construit à partir du pTG10212 (Degryse *et al*., 1995) qui est un plasmide d'expression levure basé sur un promoteur *TDH3,* un terminateur *PGK1* et un marqueur de sélection *URA3-d.*

Par recombinaison homologue dans *E. coli*, le marqueur de sélection est remplacé par le marqueur de sélection du plasmide pTG10054 (Degryse *et al*. 1995), pour ce faire le fragment *Mlu*I*, Fsp*I du pTG10054 de 2,1Kb contenant le marqueur *URA3* flanqué par des séquences de recombinaison est recombiné avec le grand fragment *Hind*III de pTG10212 pour donner le plasmide pTG10610 qui possède les mêmes caractéristiques que pTG10212 (Degryse *et al*., 1995) avec un marqueur *URA3* dans la même orientation que pTG10054.

Le fragment *Sal*I *Mlu*I portant l'ADNc du cytochrome P450 c21 humain du plasmide pTG10472 (cf. ci-dessus) est transféré dans le plasmide pTG0610 pour donner le plasmide pTG10614.

Le fragment *Cla*I *Hind*III de ce plasmide contenant de 5' vers 3', le promoteur *TDH3,* l'ADNc de la P450c21 humaine bordé par des sites *Sal*I et *Mlu*I puis le terminateur *PGK1* est transféré dans le plasmide pTG12036 pour donner le plasmide pTG12086 qui contient donc la séquence du gène *GCY1* interrompu par la cassette d'expression *TDH3* du cytochrome P450c21 humain.

### f) Construction du plasmide pTG12048.

Le plasmide pTG12048 a été construit à partir des plasmides pFL26CD, pTG10925 et pTG10953.

Le pTG10953 est identique au plasmide décrit dans Lacour *et al*., 1998.mais le promoteur *TEF1* qui est porté par un fragment *Cla*I *Sal*I est remplacé par un promoteur *CYC1* (Degryse *et al*., 1995). La cassette d'expression est portée par un fragment d'ADN *Not*I-*Not*I*.*

Le plasmide pTG10925 a été construit à partir de pFL26CD décrit par Duport *et al*., 1998. Le plasmide contient un fragment génomique de la levure qui comprend les gènes *LEU2* et *NFS1* orienté de 5' vers 3' et 3' et 5' respectivement. Une cassette d'expression de l'ADR bordé de sites *Not*I a été introduit dans la région inter-génique pour donner le plasmide pTG10925. Cette cassette (*TEF1 -* ADR bovine mature - terminateur *PGK1*) a été remplacée par une nouvelle cassette comprenant le gène *ARH1* sous contrôle du promoteur *CYC1* et du terminateur *PGK1* pour donner le plasmide pTG12048. Sur ce plasmide, le gène *LEU2* et la cassette d'expression sont dans la même orientation transcriptionnelle.

### Exemple 3. Construction de la souche TGY212#1

Une colonie est sélectionnée dans le crible décrit dans l'exemple 1. Elle est nommée TGY212#1. Cette colonie est soumise à une expérience de bioconversion comme décrit précédemment avec 100µg/ml de substrat 170H progestérone, la souche est mise à croître dans un milieu minimum complémenté avec des acides aminés nécessaires et de l'uracile.

Cette souche est capable de convertir la 170H progestérone en 11-déoxycortisol avec une efficacité de l'ordre 47% en 24 heures avec une faible production de 4 prégnène 17α,20α diol 3 one dans ces conditions (< 0.5%). Dans certaines conditions (milieu riche défini de type Kappeli avec du galactose comme source de carbone et démarrage de la culture à haute densité: D0 600nm=5), la capacité de réduction de la cétone est augmentée pour atteindre 11 % du substrat de départ avec une capacité de bioconversion réduite à 1,5% du substrat de départ.

Dans ces conditions, la présence probable du gène *GCY1* constitue une gène pour la bioconversion de la 170H progestérone. Il a donc été décidé de disrupter le gène *GCY1* pour empêcher son activité.

### Exemple 4 : Construction de la souche TGY243

Pour ce faire, la souche TGY212#1 a été transformée par 3µg du plasmide pTG12010#36 linéarisé par les enzymes de restriction *Sph*I et *EcoR*I*.* Vingt sept transformants ont été sélectionnés sur un milieu minimum complémenté pour les auxotrophies de TGY212#1 mais ne contenant pas d'uracil.

Ces colonies ont été soumises à des tests bioconversion dans un milieu minimum complémenté avec du galactose comme source de carbone car c'est un inducteur connu de *GCY1.* Tous les clones TGY243 présentaient une capacité de convertir la 170H progestérone en 11-deoxycortisol sans pour autant produire des quantités détectables de 4 prégnène 17α,20α diol 3 one.

Un clone TGY243#1 a été sélectionné pour introduire à la place du gène *URA3* une cassette d'expression pour la P450c21 humaine.

### Exemple 5 : Construction de la souche TGY245

Cette souche TGY243#1 est transformée par le plasmide YRp7 (1µg) décrit par Parent *et al*., 1985 et par le plasmide pTG12086 linéarisé par l'enzyme *Xho*I (5µg).

Le fragment transformateur de pTG12086 contient la séquence codante de *GCY1* interrompue par une cassette d'expression pour la P450c21 humaine (*TDH3:* :ADNcP450c21 humaine: terminateur *PGK1*).

Les colonies qui croissent en absence de tryptophane sont sélectionnées. Ces 3 81 colonies sont ensuite transférées sur un milieu contenant du tryptophane et du 5 fluoro orotate. Une dizaine de colonies sont ensuite testées dans un milieu riche de type YPG complémenté avec du tryptophane, de l'histidine, de la leucine et de l'uracil à une concentration de 50µg/ml.

Les souches sont mises à convertir de la 170H progestérone à une concentration de 100µg/ml à partir d'une DO600nm de 0.1 pendant 16 Heures.

Parmi ces 10 clones, un clone TGY245#2D est choisi suivant deux critères, sa capacité à convertir le 170H progestérone en 11-deoxycortisol et deuxièmement l'absence de la formation de 4 prégnène 17α,20α diol 3 one indiquant la disruption de *GCY1*.

### Exemple 6 : Construction de la souche TGY260

La souche a été construite par transformation de la TGY245 par le plasmide pTG12048 qui permet la surexpression du gène *ARH1* au locus *LEU2*

La souche TGY260 a été construite à partir de la souche TGY245 qui a été transformée par le plasmide pTG12048 linearisé.

Ce plasmide pTG12048 est un plasmide d'intégration intergènique portant le marqueur de sélection *LEU2.* Sur ce plasmide, dans la région entre le gène *LEU2* et le gène *NFS1* est intégré une cassette d'expression pour le gène *ARH1* (Lacour et *al., op. cit.*).

Cette cassette comprend le promoteur *CYC1* suivi du gène *ARH1* et du terminateur *PGK1* bordé par les sites de restriction *Not*I. Ce plasmide pTG12048 a été linearisé par les enzymes de restriction *Xho*I et *Sal*I.

Ce fragment est utilisé pour transformer la souche TGY245 en utilisant une méthode classique de transformation au chlorure de lithium. Les colonies transformantes sont ensuite sélectionnées sur un milieu ne contenant pas de leucine. Une souche TGY260 a été sélectionnée.

On a déposé la souche TGY260 à la CNCM le 24 janvier 2001 sous le numéro I-2615.

### Exemple 7 : Construction de la souche CDR07matα

La souche FY1679-28c a été transformée par le plasmide pCD62.1, décrit dans Duport *et al.* 1998, (dont le contenu technique de construction des plasmides et l'obtention des souches sont incorporés par référence à la présente demande), pour donner la souche CDR01. Celle-ci est transformée par le plasmide pCD78, également décrit dans Duport *et al*., et on obtient la souche CA03.

On disrupte le gène *ERG5* de ladite souche CA03 avec une cassette conférant la résistance à l'hygromycine, et on obtient la souche CA10.

Cette souche est croisée avec une souche parente FY1679-18b, puis mise à sporuler. Les spores sont isolées suivant les techniques classiques sur un milieu minimum complémenté pour les auxotrophies c'est à dire uracil, tryptophane, leucine et histidine.

Les spores sont criblées suivant deux critères qui sont la résistance à la nystatine (12g/ml en milieu minimum) et la résistance à l'hygromycine (100-150µg/ml en milieu riche). Les spores qui sont résistantes à ces deux produits, sont ensuite analysées en chromatographie en phase gazeuse pour la présence de campestérol comme stérol principal des membranes. La présence de campestérol dans les membranes des souches couplée à l'absence d'ergosta 5-22 dienol indique le bon fonctionnement de la Δ7 Reductase. Une spore CDR07 MATα est sélectionnée suivant les critères ci-dessus.

La souche CDR07 MATα a été déposée à la CNCM le 24 janvier 2001 sous le numéro I-2616.

### Exemple 8 : construction des souches UCY2 et UCY4

On croise les souches TGY260 et CDR07 MATα pour obtenir la souche SB14. Cette souche est alors mise à sporuler et on obtient les souches ségrégantes YCC4 et YCC5 (voir aussi ci-dessous).

Ces souches sont transformées par le plasmide pLIP5 (voir ci-dessous) et donnent respectivement les souches YCC8 et YCC9.

Ces souches sont transformées par le plasmide pTG12093 pour obtenir respectivement les souche UCY2 et UCY3.

Le gène *ATF2* est inactivé dans la souche UCY2, par utilisation d'un plasmide et d'une sélection au G418, et on obtient alors la souche UCY4.

### Exemple 9 : Construction des plasmides de transformation des souches UCY2, UCY3 et UCY4

### a) Construction du plasmide pCV29

Pour la construction du pCV29 (cf. Figure 3), les plasmides pTG10767, pTG10022 et pCD63 ont été utilisés.

### 1. Construction du plasmide pTG10767

Le plasmide pTG10767 est un plasmide d'expression pour la P450c11 d'origine humaine. Il contient en fait deux cassettes d'expression l'une pour la P450c11 d'origine humaine et l'autre pour l'adrenodoxine. Ces deux protéines sont ciblées pour être actives dans les mitochondries de la levure *S. cerevisiae,* par la présence de séquences d'adressage du précurseur de la cytochrome Cox6p.

Cette cassette d'expression pour la P450c11 d'origine humaine est bordée par deux sites de restriction *Not*I qui permettent de la transporter dans les différents vecteurs décrit par Degryse *et al*., 1995. De 5' vers 3', elle comprend, le promoteur *CYC1* comme décrit dans la publication ci-dessus puis l'ADNc de la P450c11 humaine modifié comme décrit ci-dessous et enfin une partie non codante d'eucaryote supérieur.

L'ADNc P450c11 humaine est celui décrit par Chua *et al*., 1987, avec les modifications suivantes. La partie codant pour le peptide signal dans l'ADNc original a été enlevée jusqu'au site de restriction *Bgl*II et remplacée. Cela enlève par la même occasion 31 acides aminés de la séquence codante de la forme mature de l'ADNc pour donner la séquence protéique suivante en NH2 terminal (SEQ ID N° 23) :
*MLSRAIFRNPVINRTLLRARPGAYHATRLTKNTFIQSRKY***GTRGAAAPKAVLPF EAMPRCPGNKWMRMLQI**WREQGYEDLHLEVHQTFQELGPIFRYDLGGA GMVCVMLPEDVEKLQQVDSLHPHRMSLEPWVAYRQH

Sur la ligne précédente, la partie en italique correspond à la séquence en acides aminés de la séquence d'adressage du précurseur de la cytochrome oxydase sous unité VI de levure (COX6), la partie en gras correspond à la partie NH2 terminal de la P450c11 bovine (Dumas *et al*., 1996). La partie soulignée correspond au début de la séquence identique à celle publiée de l'ADNc de la P45011β1 humaine (Kawamoto *et al*., 1990).

La cassette *Not*I qui porte le promoteur *CYC1* et l'ADNc chimérique de la P45011β1 contient également une partie non codante située en 3' de l'ADNc. Cette partie non codante est composée de 3 éléments une partie non codante provenant de l'ADNc naturel et une partie non codante provenant du plasmide pCMV4 dont la séquence est déposée à Genbank sous le numéro AF239248 (Andersson *et al*., 1989). En outre, les seules parties non codantes provenant du pCMV4 terminateur du gène de l'hormone de croissance humaine et de l'intron du gène de la β globine de lapin sont conservées dans le vecteur final pCV29.

Au final un fragment d'ADN portant des sites de restriction *Not*I contient de 5' vers 3' le promoteur *CYCI,* un ADNc chimérique encadré par des sites *Sal*I et *Mlu*I contenant une partie codant pour la préséquence de la cytochrome oxydase fusionnée à un fragment de la P450_{11β} bovine jusqu'à la partie correspondant au site de restriction puis la fin de l'ADNc correspond à l'ADNc de la P450_{11β1} humaine. La partie non codante est composée de trois d'origine de mammifère.

Cette cassette *Not*I est transférée dans le plasmide pTG10359 qui est issu du plasmide pTG10350 (Dumas *et al*., 1996) digéré par les enzymes de restriction *Cla*I et *Pvu*II et religué sur lui-même.

Ce plasmide pTG10359 possède en outre un site de restriction unique *Not*I. Le plasmide pCV29 est un plasmide 3 cassettes qui contient les cassettes d'expression pour la forme mature de l'ADX et la forme mature de la P450_{scc} et une forme de la CYP11B1 humaine ciblé à la mitochondrie.

Le plasmide pTG10022 est décrit dans la publication de Degryse *et al*., 1995, incorporée par référence à la présente demande, et contient une origine de réplication 2 micron pour la levure *S.cerevisiae* ainsi que les éléments pour le clonage dans *E. coli.*

Les sites de restriction de ce plasmide ont été modifiés avec les oligonucleotides OLIP 174 et OLIP 175 (respectivement SEQ ID N° 28 et 29) afin d'y intégrer les sites de restriction *Asc*I et *Pme*I.

Au site de restriction *Not*I de ce plasmide, on a introduit une double cassette d'expression du plasmide pCD63 décrit par Duport *et al*., 1998. Cette double cassette d'expression contenue sur un fragment *Not*I contient une cassette d'expression pour la forme mature de l'ADX bovine et une cassette d'expression pour la forme mature de la P450_{scc} bovine encadrant un marqueur de sélection *URA3.* Dans le site unique *Pme*I à bouts francs de ce plasmide, la cassette d'expression du cytochrome P45011β contenant l'ADNc de la P45011β humaine sous contrôle des promoteurs *CYC1* et terminateur *PGK1* a été introduite après remplissage des extrémités *Not*I par l'ADN polymérase fragment de Klenow.

L'un des plasmides obtenus pCV29 contient trois cassettes d'expression pour l'ADX, la P450_{scc} (toutes deux sous forme mature), ainsi qu'une forme de la P45011β humaine ciblé à la mitochondrie. En outre ce plasmide porte comme marqueur le gène *URA3* encadré par deux cassettes d'expression.

### b) Construction du plasmide pCC12

Le plasmide pCC12 (cf. Figure 4) est un plasmide réplicatif de levure à base de l'origine de réplication chromosomique comme l'*ARS1* et d'un centromère *CEN* (il y en a 16 dans la levure) tous les deux de *S. cerevisiae*. Ce plasmide se réplique sous forme à une ou deux copies dans la levure.

Il a été construit à partir du pFL38C2 qui est dérivé de pFL38 (Bonneaud *et al*., 1991, dont le contenu est incorporé par référence à la présente demande, notamment les descriptions des plasmides). Dans ce plasmide, on a introduit au site *Hind*III un oligonucléotide qui contient la séquence de reconnaissance des sites *Not*I, *Pac*I et *Asc*I (OLIP FL SEQ ID N° 24). L'orientation du polylinker a été vérifiée par séquençage. Ce plasmide porte un marqueur de sélection *URA3* bordé par des sites *Bgl*II.

Le gène *URA3* a été remplacé par un gène *ADE2* de 2,7Kb obtenu par amplication par PCR sur le génome de FY1679-28c en utilisant les oligonucléotides 5'ADE2090 (SEQ ID N° 37, CGATTCGGATCCACTAGTAACGCCGTATC-GTGATTAACG) et 3'ADE2089 (SEQ ID N° 38 (CCTCAAGGATCCTCCTG-ACGTAGCGCTATCCTCGGTTCTG).

Pour ce faire le plasmide pFL38C2 a été digéré par l'enzyme *Bgl*II, le fragment *Bgl*II *URA3* a été remplacé par le fragment de 2,7 Kb contenant le gène *ADE2.* Le plasmide obtenu pAM1 sert de base pour la construction du plasmide pCC12.

Construction du plasmide à partir du plasmide pAM1 :

Le plasmide pCC12 contient deux cassettes d'expression l'une pour le 3β-HSDH d'origine bovine l'autre pour l'adrenodoxine reductase également d'origine bovine.

L'ADNc de la 3β-HSDH bovine a été recloné par PCR à partir d'une banque d'ADNc de surrénales de boeuf. La séquence codante de l'ADNc publié par Zhao *et al*., 1989, n'a pas été modifiée.

Les deux oligonucléotides rajoutent un site *Sal*I et 4 adénines devant l'ATG pour donner la séquence GTCGACAAAAATG (SEQ ID N° 25). Le site *Mlu*I est situé au ras du codon de terminaison de l'ADNc de la 3β-HSDH bovine pour donner la séquence: fin de l'ADNc TGACCTGGAGTGACAATGACGCGT (SEQ ID N° 26). La séquence reconnue par l'enzyme *Mlu*I est ACGCGT.

L'ADNc est transféré sous forme d'un fragment *Sal*I, *Mlu*I dans le plasmide pTG10212 pour donner le plasmide pCC4. Ce plasmide possède donc un fragment *Not*I contenant l'ADNc de la 3β-HSDH bordé par et un promoteur *TDH3* et un terminateur *PGK1*, respectivement en 5' et en 3'. Cette cassette d'expression est transférée ensuite dans le plasmide pTG12018 ainsi la cassette est maintenant bordée en 5' d'un site *Not*I et en 3' d'un site *Asc*I. Ce fragment est ensuite cloné dans le plasmide d'expression de la levure pAM1 dans les sites *Not*I et *Asc*I pour donner le plasmide pCC11.

Dans le site *Not*I de ce plasmide, on insère le fragment *Not*I portant le promoteur *TEF1,* l'ADNc de la forme mature de l'adrenodoxine reductase d'origine bovine, et le terminateur de levure *PGK1,* respectivement dans cet ordre provenant du plasmide pTG10361.

Ce plasmide porte le même ADNc qui est décrit dans Dumas *et al*., 1996, (dont le contenu est incorporé par référence à la présente demande, notamment les descriptions des plasmides) sauf que la séquence d'adressage du précurseur de la cytochrome oxydase a été remplacée par un codon methionine. L'ADNc code donc pour une forme mature de l'ADNc de l'adrenodoxine reductase.

### Exemple 10 : Construction de la souche CDR07

La souche CDR07 a été obtenue par croisement entre les souches FY1679-18b MAT a et la souche CA10 MAT a décrite par Duport *et al*., 1998.

Ces deux souches ont été tout d'abord isolées sur un milieu riche contenant du glycérol puis sur un milieu dans un milieu contenant de l'acétate de potassium comme décrit dans « Yeast Protocols Methods in Cell and Molecular Biology Edited By Ivor H Evans in 1996. Humana Press Totowa New Jersey ».

Après sporulation, les tétrades ont été digérées par de la zymolyase 100T pendant 30 minutes à 37°C. Plusieurs cycles de sélection ont été appliqués pour obtenir un clone qui produise du campéstérol comme stérol majoritaire en ayant perdu les autres caractères de CA10.

Les spores sont tout d'abord sélectionnées sur un milieu minimum contenant de la nystatine avec des suppléments (adénine, leucine, tryptophane, uracile, histidine). Les clones résistants à la nystatine et auxotrophe pour l'adénine et la leucine sont ensuite repiqués sur milieu riche contenant de l'adénine et d'hygromycine (200µg/ml) pour détecter la délétion du gène *ERG5* par le gène de résistance à l'hygromycine.

Des clones auxotrophes pour l'adénine, la leucine (et l'uracile et le tryptophane) et également résistants à l'hygromycine et à la nystatine sont analysés pour leur profil stérolique. Deux clones de signes sexuels opposés produisant du campéstérol comme stérol majoritaire sont sélectionnés.

Ils sont nommés CDR07 MAT a et CDRO7 MAT α.

### Exemple 11 : Construction des plasmides d'intégration pTG12093 et pLIP5 respectivement pour les locus HIS3 et TRP1.

### a) Construction du pLIP5

Le pLIP5 est plasmide qui peut être utilisé pour les intégrations génomiques au locus *TRP1* ou comme plasmide multicopie dans la levure *S.cerevisiae.* Le plasmide de base qui a servi à la construction de ce plasmide est le plasmide pFL45S décrit dans Bonneaud *et al.* 1991, dont le contenu est incorporé par référence à la présente demande, notamment les descriptions des plasmides).

Un fragment d'ADN génomique correspondant à la partie 5' en amont du promoteur *TRP1* a d'abord été cloné en utilisant les oligonucléotides OLIP21 et OLIP22 (respectivement SEQ ID N °30 et 31) pour faire une amplification par PCR.

Le produit de PCR a d'abord été sous cloné dans pCR-Script AmpSK(+) (Stratagène, La Jolla California USA). Les extrémités OLIP21 et OLIP22 possèdent en effet en 5' un site *Nar*I ainsi qu'un site *Hind*III en 3'. Ce fragment *Nar*I, *Hind*III a remplacé les sites de clonage multiples du plasmide pFL45S décrit plus haut.

Le plasmide obtenu pLIP3 est de nouveau modifié en utilisant l'oligonucléotide OLIP20 (SEQ ID N° 32) qui sert à remplacer le site unique *Hind*III par le site unique *Not*I. Dans ce site *Not*I, on introduit un fragment contenant de 5' vers 3' le promoteur *TEF1,* l'ADNc du cytochrome P-450c17alpha puis le terminateur *PGK1* comme décrit dans Degryse *et al*., 1999 (dont le contenu est incorporé par référence à la présente demande, notamment les descriptions des plasmides).

Le plasmide final pLIP5 peut servir à la fois de plasmide multicopie à base du marqueur *TRP1,* lorsque la partie 2 micron est retirée, on peut l'utiliser comme plasmide d'intégration au niveau du locus *TRP1.* La cassette est ainsi intégrée en 5' du gène *TRP1.*

### b) Construction du plasmide pTG12093

Ce plasmide est un plasmide d'intégration intergénique au niveau du locus *HIS3.* Ce plasmide est construit à partir du plasmide pUC-HIS3 décrit par Duport *et al*., 1998. Le site unique de restriction *Xho*I de ce plasmide a été transformé en un site de restriction unique *Not*I en utilisant un adaptateur approprié tout en détruisant le site *Xho*I pour donner le plasmide pUC19-*HIS3*. Dans le site *Not*I unique de ce plasmide, un fragment *Not*I provenant du pTG10792. Ce fragment contient le promoteur *TDH3* l'ADNc codant pour l'ADX bovine mature fusionnée à la séquence d'adressage du précurseur de la cytochrome de COX₆ ( de la sous unité 6 de la cytochrome oxidase de levure comme décrit dans Dumas *et al*., 1996. Le fragment de restriction *Sal*I *Mlu*I du plasmide pTG10350 contenant l'ADNc précédemment décrit a été transféré dans le plasmide pTG10211 pour former le plasmide pTG10792.

Ce plasmide possède donc un fragment de 1,6 kilobases contenant le promoteur *TDH3,* l'ADNc fusionné entre l'ADX bovine mature et la présequence COX₆ de levure, et le terminateur *PGK1* de 5' vers 3'. Ce fragment *Not*I-*Not*I est inséré dans le plasmide pTG12093. La transcription de *HIS3* et de la cassette d'expression sont dans la même orientation.

### Exemple 12 : Croisement de CDR07 MATα avec TGY260 MATa

La souche SB 14 (CDR07MATα X TGY260 MATa) est mise à sporuler dans un milieu très pauvre contenant de l'acétate de potassium. Les différents ascii sont ensuite mise à pousser dans un milieu minimum contenant les produits suivant uracile, histidine, tryptophane et adénine.

Les spores sont ensuite sélectionnées sur un milieu minimum correctement complémenté contenant de 8 à 12µg/ml de nystatine. Les spores positives sont ensuite sélectionnées sur la présence de l'ADNc de la P450-c21 humaine. Pour ce faire, on réalise un criblage des clones en milieu Kappeli (Arreguin de Lorencez M, Kappeli O J, 1987) avec comme source de carbone 2% d'éthanol (et 0,1% glucose) en présence de 300mg/ml de 170H progestérone.

La bioconversion est incubée jusqu'à 72 heures. La capacité de bioconversion est analysé en HPLC comme décrit par Dumas *et al*., 1996 et Degryse *et al*., 1999 (dont les contenus sont incorporés par référence à la présente demande, notamment les explications des études de bioconversion).

Ces clones sont également analysés pour leur profil stérolique en chromatographie en phase gazeuse comme décrit par Duport *et al*., 1998 (dont le contenu est incorporé par référence à la présente demande, notamment les explications des études de bioconversion) en vue de détecter le campestérol et l'ergosta 5,22 diénol.

Trois spores YCC3, YCC4 et YCC5 sont sélectionnées comme produisant de l'ergosta 5,22 diénol et du campéstérol et convertissant le 170H-progestérone en 11-deoxycortisol avec une efficacité de production de 25, 120 et 42 µg/ml en 72 heures, respectivement.

YCC4 et YCC5 sont ensuite sélectionnés pour deux nouvelles transformations successives avec les plasmides pLIP5 et pTG12093 linéarisés respectivement par les enzymes ApaL1 et EcoR1. Les plasmides pLIP5 et pTG12093 sont des plasmides d'expression intergénique respectivement pour la P450c17 bovine et l'ADX bovine mitochondriale.

Le pTG12093 est un plasmide d'intégration dans la région intergénique en 3' du locus HIS3 tandis que le pLIP5 est un plasmide d'intégration dans la région intergénique en 5' du locus *TRP1.* En outre ces deux plasmides portent un site unique *Not*I qui permet l'intégration d'une cassette d'expression contenant dans cet ordre; promoteur *TEF1,* ADNc P450c17 bovine, terminateur *PGK1* pour le pLIP5 dans cette ordre promoteur *TDH3,* ADNc COXVIpre: :mat ADX bovine, terminateur *PGK1* pour le pTG12093.

La souche YCC4 est transformée successivement par les plasmides pLIP5 et pTG12093 linéarisés (par les enzymes de restriction *ApaL*I et *EcoR*I).

Lors de la première transformation, les clones transformants sont tout d'abord sélectionnés sur un milieu ne contenant pas de tryptophane. Les clones qui poussent en l'absence de tryptophane sont ensuite sélectionnés par PCR avec les oligonucléotides C17-3 et C17-5 (respectivement SEQ ID N° 33 et SEQ ID N° 34).

Un clone YCC8 est sélectionné pour une nouvelle transformation par le plasmide pTG12093 linéarisé. De la même manière, les clones qui poussent en l'absence d'histidine sont sélectionnés puis la présence de l'ADNc ADX est vérifiée par PCR à l'aide des oligonucléotides ADX-3 ADX-5 (respectivement SEQ ID N° 35 et SEQ ID N° 36).

Un clone UCY2 est sélectionné, son signe sexuel est MATα.

### Exemple 13 : Construction de la souche UCY4

La souche UCY2 possède un gène *ATF2* fonctionnel c'est à dire que la plupart de la pregnenolone produite est transformée en acétate de pregnenolone par la protéine ATF2p qui est une pregnenolone acétyl transférase dont la fonction naturel dans la levure est inconnue (Cauet *et al*., 1999). De plus cette réaction est irréversible et donc, une fois produite l'acétyl pregnenolone n'est plus transformable en hydrocortisone. Il apparaît donc important détruire cette activité pour permettre une meilleure production d'hydrocortisone.

Le gène *ATF2* a donc été disrupté en utilisant un gène de résistance au G418. Pour ce faire un plasmide pAM3kanaC de disruption a été construit permettant l'introduction d'un marqueur de résistance au G418 dans le gène *ATF2.*

Ce plasmide a été construit à partir du plasmide pAM1 (dont la construction a été décrite plus haut), du plasmide pTG12002 qui est un plasmide d'expression pour le gène *ATF2.*

Le pTG12002 est un plasmide d'expression pour *ATF2* basé sur le plasmide pTG10260 (Degryse *et al*., 1995) dans le quel le site de restriction *Xba*I de l'origine de réplication 2 micron a été inactivé. Ce plasmide comprend donc une cassette d'expression pour le gène *ATF2* (Cauet *et al*., 1999) comprenant le promoteur *CYC1,* le gène *ATF2* (encadré par les sites de restriction *Sal*I et *Mlu*I) et le terminateur *PGK1.* Cette cassette a été modifiée par PCR pour contenir le gène *ATF2* complet comprenant le promoteur du gène *ATF2,* la séquence codante du gène *ATF2,* le terminateur du gène *ATF2* sur un fragment de restriction *Kpn*I, *Not*I. Ce fragment *Kpn*I-*Not*I est introduit aux sites *Kpn*I-*Not*I du plasmide pAM1 pour donner le plasmide pAM3.

Dans le plasmide pAM3, la cassette d'expression de la résistance au G418 est introduite dans le gène *ATF2* entraînant son inactivation.

Pour ce faire le plasmide pAM3 est digéré par l'enzyme de restriction *Acc*I, puis partiellement par l'enzyme de restriction *Sac*I. Une bande d'environ 7500pb est purifiée sur gel. Le plasmide pFA6a kanMX4 (Wach et al Methods in Microbiology Volume 26 Yeast Gene Analysis Chapitre 5 PCR-based Gene Targeting in Saccharomyces cerevisiae) est digéré par *Sac*I et *Acc*I, la bande à 1500pb est purifiée sur gel. Les deux fragments sont ligués puis transformés. Un plasmide est obtenu pAM3kanaC. Le pAM3kanaC est digéré par *Pvu*II et *Not*I, la bande à 2215pb est purifiée sur gel, puis transformé dans UCY2 qui est étalée sur des boites de milieu riche contenant 130 µg/ml de G418. Environ 600 clones sont repiqués sur ce milieu contenant du G418, deux clones sont résistant au G418. Un clone ne contenant pas le plasmide pAM3 est conservé.

Cette méthode d'inactivation de gène est bien connue de l'homme du métier.

Le clone unique ainsi obtenu est mis à bioconvertir avec 100µg/ml de pregnénolone dans du milieu Kappeli.

Au bout de 24 heures, l'absence de pregnenolone acétate est vérifié par extraction et chromatographie en phase gazeuse comme décrit dans Cauet *et al* 1999. Ce phénomène indique que le gène *ATF2* responsable de l'acétylation de la pregnenolone a bien été disrupté et n'est plus fonctionnel. La souche est nommée UCY4.

### Exemple 14 : Construction des souches UCY3 et UCY26.

Le criblage des spores obtenues par croisement des souches CDR07 et TGY260 a donné plusieurs souches dont les souches YCC4 et YCC5. Comme décrit ci-dessus, la souche YCC4 a été transformée par une série de 2 plasmides : pLIP5 et pTG12093. Une spore UCY2 a alors été sélectionnée (cf. exemple 12). De la même manière, la souche YCC5 a également été transformée par les plasmides pLIP5 et pTG12093 et une nouvelle spore nommée UCY3 a été obtenue. Comme UCY2, UCY3 est caractérisée par la présence et l'expression de la Δ7 réductase d'*A. thaliana* mesurée par la résistarice à la nystatine et la présence de brassicastérol et de campestérol comme stérol majoritaire de ces levures recombinantes. La présence de l'ADNc ADX est vérifiée par PCR à l'aide des oligonucléotides ADX-3 et ADX-5 (respectivement SEQ ID N° 35 et SEQ ID N° 36), puis par Western blot comme décrit dans Dumas *et al*., 1996, incorporé par référence ici pour ce qui est de la description de cette technique.

L'activité d'hydroxylation en 17 et en 21 de la progestérone est vérifiée par bioconversion de la progestérone en 17OH-progestérone est 11-deoxycortisol. Pour ce faire, la souche est incubée en présence de 200mg/l de progestérone comme décrit dans Dumas *et al*., 1996 et Degryse *et al*., 1999 UCY3 est capable de produire du 11-deoxycortisol à partir de la progestérone indiquant la présence des activités P450c17 et P450c21. On détecte également la présence de 4 prégnène 17α,20α diol 3 one indiquant qu'au moins une des deux activités codées par GCY1 ou YPR1 est présente dans la souche. Pour éviter l'accumulation d'acétate de pregnenolone, l'activité d'acétylation de la pregnenolone codée par le gène *ATF2* a été éliminée. Dans ce but, le plasmide pAM3kanaC (cf. exemple 13) a été utilisé pour transformer la souche UCY3. Le pAM3kanaC est tout d'abord digéré par *Pvu*II et *Not*I, la bande à 2215pb est purifiée sur gel, puis transformée dans UCY3 qui est étalée sur des boites de milieu riche contenant 130 µg/ml de G418. Des colonies résistantes à l'antibiotique G418 et qui ne sont plus capables de transformer la pregnenolone en acétate de pregnenolone sont isolées ; une colonie UCY26 est plus particulièrement sélectionnée pour de nouvelles transformations et pour tester sa production d'hydrocortisone.

### Exemple 15 : Construction de la souche UCY5.

Dans le but de disposer d'une meilleure variabilité génétique, la souche UYC2 (cf. exemple 12) a été croisée avec la souche TGY245 (cf. exemple 5), une souche diploïde YSA2-2n a ainsi été sélectionnée. Cette souche a été mise en condition de production d'asques et 85 asques ont été disséqués (comme décrit dans « Yeast Protocols in Molecular Biology Volume 53 p59-67, 1996 »). Les spores isolées sont criblées sur leur propriété auxotrophe. Ainsi, les clones capables de croître en l'absence de tryptophane et d'histidine et nécessitant de l'adénine sont plus particulièrement sélectionnés. L'expression de la Δ7 réductase est vérifiée en s'assurant de la résistance de la souche à la nystatine ainsi que de la présence de campestérol et brassicastérol dans la membrane de ces souches. Cette dernière analyse est réalisée par chromatographie en phase gazeuse des stérols totaux de ces souches comme décrit dans Duport et al., 1998. De plus la présence des ADNcs codant pour la P450c17 et l'ADX est vérifiée par PCR à l'aide des oligonucléotides C17-3 et C17-5 (respectivement SEQ ID N° 33 et SEQ ID N° 34) et des oligonucléotides ADX-3 et ADX-5 (respectivement SEQ ID N° 35 et SEQ ID N° 36) respectivement. Finalement, la fonctionnalité des gènes GCY1 et YPR1 dans ces spores positifs est vérifiée de deux manières : soit par PCR, soit par absence de l'activité 20 alpha réductase sur la 17 OH progestérone.

Par PCR, en utilisant la paire d' oligonucléotides X1TEF1 et X2C21 (respectivement SEQ ID N° 47 et SEQ ID N° 48), on détecte la disruption du gène YPR1 par la cassette d'expression pour la P450c21 humaine dans *S.cerev*isiae contenant le promoteur TEF1, le cDNA de la P450c21 humaine ainsi que le terminateur PGK. On détecte également par PCR, en utilisant la paires d'oligonucléotides X3TDH3 et X2C21 (respectivement SEQ ID N° 49 et SEQ ID N° 48), la disruption du gène GCY1 par la cassette d'expression pour la P450c21 humaine dans *S.cerevisiae* contenant le promoteur TDH3, le cDNA de la P450c21 humaine ainsi que le terminateur PGK.

La disparition des deux activités GCY1 et YPR1 et la présence de l'activité correspondant à l'activité P450c17 et P450c21 sont finalement vérifiées par bioconversion de la progestérone en 11-deoxycortisol dans les conditions décrites par Dumas et al 1996 modifiées par Degryse et al 1999. Les levures recombinantes sont incubées à 30°C en présence de 200mg/l de progestérone avec une densité cellulaire de 5 dans un milieu de culture de type Kappëli contenant 2% de galactose ou 2% de glucose comme source de carbone, dans un volume de 10ml. Après une incubation de 48 heures, on extrait le milieu de culture de ces levures comme décrit précédemment et on mesure en particulier la quantité de 17OH progestérone et 11-deoxycortisol produite par HPLC. La spore sélectionnée ne produit plus de quantité détectable de 4 prégnène 17α,20α diol 3 one mais produit de la 17OH progestérone et du 11-deoxycortisol en utilisant les deux sources de carbone. Le clone choisi produit la plus grande quantité de 11-deoxycortisol.

Une souche répondant positivement à tous ces critères est plus particulièrement sélectionnée pour de nouvelles transformations, cette souche est nommée UCY5.

### Exemple 16 : Construction des plasmides pFM10 et pTG10897.

### a) Construction du plasmide pFM10

Le plasmide pFM10 est un vecteur permettant l'expression simultanée de 4 protéines chez la levure. Il ne contient pas d'origine de réplication pour *E*. *coli* ni de gène de résistance à l'ampicilline, ce plasmide ne peut donc pas se répliquer chez *E.coli.* Son obtention est réalisée par recombinaison chez la levure de deux plasmides : pFM7 et pCB12. Contrairement au plasmide pFM10, ces derniers se répliquent tous deux chez *E. coli* puisqu'ils possèdent le réplicon *E.coli.* Le plasmide pFM7 se réplique également chez *S.cerevisiae,* puisqu'il comporte également, l'origine de réplication 2 micron de la levure *S.cerevisiae.* Ces deux plasmides possèdent en outre les séquences R1 et R2 (respectivement SEQ ID N° 39 et SEQ ID N° 40) bordant les cassettes d'expression ainsi que les marqueurs de sélection. Les séquences R1 et R2 proviennent du gène de la photochlorophyllide oxydoréductase *d'A thaliana* et leur taille est d'environ 300 paires de bases chacune.

Les deux séquences R1 et R2 sont clonées en orientation inverse dans les plasmides pFM7 et pCB12. Ainsi, entre les séquences R1 et R2, le plasmide pFM7 contient dans l'ordre : l'origine de réplication 2 micron contenu dans le fragment 2 micron (fragment bordé par les sites de restriction EcoRI, tel que décrit par Urban *et al*., 1994), un fragment bordé par des sites *Not*I provenant du plasmide pCD63 décrit dans Duport *et al*., 1998 contenant deux cassettes d'expression pour la forme mature de la P450_{scc} et la forme mature de l'ADX séparée par le gène *URA3* de la levure fonctionnel, vient ensuite la séquence R2. De la même façon le plasmide pCB12 contient les séquences R1 et R2 mais clonées dans le sens inverse de celle du plasmide pFM7. Ainsi, entre les séquences R2 et R1, on trouve la cassette d'expression pour la P450_{11β}, le marqueur de sélection ADE2, la cassette d'expression pour la 3β-HSD d'origine bovine. La cassette d'expression pour la P450_{11β} provient du plasmide pCV29 et contient le promoteur CYC1, un ADNc hybride codant pour la P450_{11β} et un terminateur PGK. Le gène ADE2 provient du plasmide pAM1, il s'agit du fragment *BglII-BglII.* De même, la cassette d'expression pour la 3β-HSD bovine c'est à dire le promoteur TDH3, l'ADNc de la 3β-HSD bovine et le terminateur PGK provient du plasmide pCC12, il s'agit du fragment *C*/*aI-AscI.*

### b) Construction du plasmide pTG10897

A l'aide de deux oligonucléotides 20mers correspondant respectivement à la partie 5' et 3' de la séquence codante du gène *ATF2* (et comprenant également les sites de clonage) et en utilisant comme matrice de l'ADN génomique de la souche FY 1679-28c, on amplifie par PCR la séquence codante du gène *ATF2.* Ce produit de PCR est ensuite digéré par les enzymes *ClaI* et *HindIII* et est cloné entre les sites *ClaI* et *HindIII* du plasmide pTG10031 [Degryse, 1995 #102] pour donner le plasmide pTG10885. Ce plasmide sert de base à la construction d'un plasmide de disruption du gène *ATF2.* La séquence du gène *URA3* a été amplifiée à partir d'ADN génomique de la souche TGY156 (décrit dans Cauet *et al*. 1999 et incorporé ici par référence), cette souche possède en effet un gène ATF2 interrompu par le gène URA3. Les oligonucléotides OTG10842 (SEQ ID N° 41) et OTG10841 (SEQ ID N° 42) ont donc servi à l'amplification sur matrice d'ADN génomique de la souche TGY156 et le produit de l'amplification a servi d'amorce de recombinaison avec le plasmide pTG10885 digéré par les enzymes de restriction *BstXI* et *StuI.*
On obtient ainsi le plasmide pTG10897 contenant la séquence codante du gène *ATF2* interrompue par le gène *URA3.* En effet, le gène URA3 fonctionnel se trouve entre 509 nucléotides de la partie 5' de la séquence codante d'*ATF2* et 444 nucléotides de la partie 3'de cette séquence codante.

### Exemple 17 : Construction des souches UCY6 UCY16, UCY16-pFM10, UCY19, UCY20, UCY24, UCY25, UCY27.

A partir de la souche UCY5, une série de nouvelles souches a été construite dans le but d'améliorer la production de stéroïde d'intérêt. En effet, la souche UCY5 n'exprime pas d'adrenodoxine réductase qui est un composant essentiel de la réaction de coupure de la chaîne latérale par la P450_{scc}. D'autre part, comme il a été décrit auparavant, le gène *ATF2* code pour une acétyl transférase qui utilise la prégnénolone comme substrat et sa disruption permet de supprimer cette réaction parasite d'acétylation et d'augmenter ainsi le rendement en stéroïde d'intérêt. Enfin, la voie de biosynthèse exogène utilise l'activité ARH1p endogène, cette dernière étant essentielle à la survie de la levure. Cependant, cette activité mitochondriale, qui remplace l'adrenodoxine réductase de mammifère, pourrait être limitante dans les souches produisant de l'hydrocortisone. Une souche possédant certaines modifications permettant d'augmenter de manière conséquente le rendement de production en stéroïde d'intérêt a donc été construite à partir de la souche UCY5,. Ainsi cette souche est dépourvue d'activité ATF2, surproduit la protéine adrenodoxine réductase et surproduit également la protéine ARH1.

Pour permettre une expression de l'ADR dans la souche UCY5, cette dernière a été transformée par le vecteur pTG10925 (cf. exemple 2 f) qui lorsqu'il est transformé dans la levure sous forme linéaire permet l'intégration au locus *LEU2* et l'expression de l'ADR sous contrôle du promoteur *TEF1.* L'expression de l'ADR a été vérifiée par Western blot comme décrit dans Dumas *et al*., 1996. Un clone exprimant l'ADR et nommé UCY6 a plus particulièrement été utilisé pour les transformations suivantes.

Dans le but d'éliminer la réaction parasite d'acétylation transformant la prégnenolone en acétate de pregnenolone et catalysée par l'enzyme ATF2, le gène *ATF2* codant pour cette enzyme a été disrupté. Ce dernier a en effet été interrompu par le marqueur *URA3*, pour ce faire, le fragment *Not*I du plasmide pTG 10897 qui contient la séquence du gène *ATF2* interrompu par le gène fonctionnel de levure *URA3* a été utilisé. Ce fragment est utilisé pour transformer la souche UCY6. Les colonies capables de croître en l'absence d'uracile sont sélectionnées, puis on mesure la capacité de ces clones à transformer la pregnenolone en acétate de pregnenolone comme décrit par Cauet et al., 1999. Un clone capable de croître en l'absence d'uracile et incapable de transformer la pregnenolone en acétate de prégnenolone est plus particulièrement isolé. Ce clone est appelé UCY16.

Cette souche ne pouvant pas être transformée par des plasmides portant uniquement le marqueur *URA3*, deux nouveaux plasmides ont été construits: les plasmide pCB12 et pFM7. Ces deux plasmides lorsqu'ils sont recombinés ensemble, permettent d'obtenir le plasmide pFM10 basé sur les marqueurs *URA3* ou/et *ADE2* (cf. ci-dessus et Figure 5). Ainsi pour obtenir le plasmide pFM10, le plasmide pFM7 est linéarisé par l'enzyme de restriction *AatII* et le fragment d'ADN correspondant est isolé sur gel. Le plasmide pCB12 est quant a lui digéré par *BamH1* et la bande de 9300 paires de bases est isolée selon les techniques classiques de biologie moléculaire. Environ 5 µg des fragments de pCB12 et pFM7 sont mélangés et servent à transformer la souche UCY16. Quelques clones UCY16-pFM10, capables de pousser en milieu minimum (sans uracile et d'acides aminés), sont isolés et les souches correspondantes sont testées pour leur niveau de production de stéroïdes selon le protocole décrit ci-dessous.

Alternativement et dans le but de pouvoir utiliser par la suite les plasmides du type pCV29 basés sur un marqueur *URA3,* la disruption du gène *ATF2* dans la souche UCY6 a également été obtenue en transformant cette souche par le plasmide pAM3kanaC linéarisé (cf. exemple 13). Les colonies résistantes au G418 sont ensuite testées pour l'absence ou la présence de l'activité pregnenolone acétyl transférase comme décrit par Cauet *et al.* 1999. Une colonie résistante au G418 et ne possédant plus l'activité pregnenolone acétyl transférase est plus particulièrement sélectionnée. Cette souche est appelée UCY24.

Dans le but d'augmenter l'activité ARH1 dans une souche contenant une voie de production exogène de l'hydrocortisone, la souche UCY5 a été transformée par les plasmides pTG12048 ou pTG12050 linéarisé au préalable par *XhoI* et *SapI* respectivement. Le plasmide pTG12048 qui a été décrit ci-dessus permet la surexpression du gène *ARH1* au locus *LEU2* sous contrôle du promoteur *CYC1.* Le plasmide pTG12050 diffère du plasmide pTG12048 uniquement par le fait que le promoteur *CYC1* contrôlant l'expression du gène *ARH1* a été remplacé par le promoteur *TEF1* tel que décrit dans Degryse *et al*., 1995 et Dumas *et al.* 1996. UCY5 a été transformée par pTG12048 ou pTG12050 linéarisé. Dans chacun des deux cas, les colonies capables de pousser en l'absence de leucine ont été sélectionnées. De plus la présence des cassettes d'expression pour le gène ARH1 a été vérifiée par PCR. Deux couples d'oligonucleotides permettent en effet de vérifier la présence d'une copie supplémentaire du gène *ARH1* (sous contrôle du promoteur *CYC1* ou *TEF1*). D'une part, le couple arh1D (SEQ ID N° 43) et nfs1R (SEQ ID N° 44) permet de vérifier la présence de la jonction entre le gène *ARH1* et le gène *NFS1.* D'autre part, le couple leu2D (SEQ ID N° 45) et arh1R (SEQ ID N° 46) permet la vérification de la jonction entre le gène *LEU2* et le gène *ARH1.*

Outre la présence de la cassettes d'expression pour le gène ARH1 codant pour l'activité ARH1, l'expression de la protéine ARH1p a également été testée dans les clones obtenus. La détection de la surexpression d'ARH1p n'est cependant pas chose aisée. Ce fait est dû à la présence naturelle de la protéine ARH1p à un faible niveau dans les souches de *S.cerevisiae.* Des expériences de Western Blot réalisées comme décrit dans Dumas *et al*., 1996 permettent cependant de confirmer l'augmentation de la quantité d'ARH1p. En plus des expériences de Western Blot qui s'avèrent délicates, la présence d'une quantité accrue d'ARH1p peut être vérifiée par des expériences de réduction du cytcochrome c ou de 11beta hydroxylation du 11-deoxycortisol reconstituées *in vitro* avec des mitochondries purifiées des levures recombinantes comme décrites dans Lacour *et al*., 1998 et Dumas *et al*., 1996, respectivement. Deux clones, UCY19 et UCY20, respectivement transformé par pTG12048 et pTG12050 et surexprimant *ARH1*, sont plus particulièrement sélectionnés. Le gène *ATF2* a ensuite été disrupté dans ces souches comme décrit plus haut. Brièvement, le plasmide pAM3kanaC a été linéarisé puis utilisé pour transformer les souches UCY19 et UCY20. Les clones ont ensuite été sélectionnés pour leur propriété de résistance au G418 et l'absence d'activité pregnenolone acétyl transférase comme décrit par Cauet *et al*. 1999. Deux clones dérivés de UCY19 et UCY20 sont plus particulièrement sélectionnés. Il s'agit respectivement des souches UCY25 et UCY27 (cf. Figure 2).

### Exemple 18 : Production de stéroïdes par les souches selon l'invention

### a) Transformation des UCY2 et UCY4 par pCV29 et pCC12

Les souches UCY2 et UCY4 ont été transformées par les deux plasmides pCV29 et pCC12, décrits plus haut, dont les structures sont rappelées ci-dessous et dans les figures 3 et 4.

Le plasmide pCV29 porte une origine de réplication de type 2 micron pour réplication chez la levure. En outre, ce plasmide porte 3 cassettes d'expression respectivement pour la P45011β humaine/bovine hybride ciblé à la mitochondrie tel que décrit précédemment, les formes matures (avec une méthionine à l'extrémité NH2 terminal) de l'adrenodoxin et de la P450_{scc}. L'expression de ces trois protéines est sous le contrôle des promoteurs *CYC1* (P45011β) et *GAL10*/*CYC1* (ADX et P450_{scc} sous forme mature ).

Le plasmide pCC12 est un plasmide à bas nombre de copie qui porte une cassette d'expression pour la forme mature de l'adrenodoxin reductase bovine sous contrôle du promoteur *TEF1* ainsi qu'une cassette d'expression pour la 3β-HSD bovine sous contrôle du promoteur *TDH3.*

Ces souches sont mises à pousser dans des conditions classiques de fermentation en milieu Kappeli avec du glucose comme source de carbone. A la fin de la fermentation (180h), les stéroides sont extraits comme décrit précédemment et caractérisés en chromatographie liquide haute pression.

L'identité des produits a été vérifiée par chromatographie en phase gazeuse, par chromatographie haute pression en phase liquide et en phase reverse couplé ou
non avec la spectrométrie de masse et la résonance magnétique nucléaire (cf. Table 1).

**Table 1. Bilan des stéroïdes obtenus (résultats exprimés en mg/l)**

| Souches | Acétate de prégnénolone | Progestérone | 4-prégnène-17α-20α diol 3 one | Non-identifié | Corticostérone | Hydrocortisone |
|---|---|---|---|---|---|---|
| UCY2/pCV29 + pCC12 | 18 | 7 | 5 | 5 | 12 | 15 |
| UCY4/pCV29 + pCC12 | 0 | 30 | 44 | 24 | 23 | 80 |

### b) Transformation des souches UCY24, UCY25, UCY26 et UCY27 par pCV29 et pCC12 et transformation de la souche UCY16 par pFM10

Ces souches possèdent de nombreuses modifications et s'avèrent difficiles à transformer. En conséquence, un protocole basé sur la préparation de sphéroplastes a été utilisé pour ces transformations, comme décrit par Burgers *et al*., 1987. UCY16 a été transformé suivant ce protocole par les plasmides pFM7 et pCB12, ces derniers donnant le plasmide pMF10 par recombinaison *in vivo* chez la levure. Comme décrit ci-dessus, le plasmide pFM10 est un plasmide multicopie de levure, ne contenant pas de séquences bactériennes et permettant l'expression de 4 protéines hétérologues. Ces quatre protéines sont comme vu précedemment: une forme chimérique du cytochrome P450_{11β}, la 3β-HSD bovine, l'ADX mature et le cytcochrome P450_{scc} mature. Les ADNc correspondants sont placés respectivement sous le contrôle des promoteurs *CYC1, TDH3, GAL10*/*CYC1* et *GAL10*/*CYC1.* Les transformants sont sélectionnés sur un milieu minimum sans aucun ajout. La sélection se fait uniquement sur le marqueur *ADE2* (croissance en l'absence d'adénine), la présence d'un gène fonctionnel *URA3* dans le génome et qui sert à l'inactivation du gène *ATF2* ne permet pas la sélection sur le gène *URA3.* Il est donc nécessaire de cribler plusieurs colonies pour être sûr d'avoir un plasmide pFM10 fonctionnel dans la souche UCY16..

Les souches UCY24, UCY25, UCY26 et UCY27 ont été transformées par les plasmides pCV29 et pCC12 selon le même protocole de préparation de sphéroplastes. Comme décrit ci-dessus, le plasmide pCV29 est un plasmides multicopie navette entre *E.coli* et *S.cerevisiae*. Il permet l'expression du cytochrome P450_{11β}, l'ADX mature et du cytochrome P450_{scc} respectivement sous le contrôle des promoteur *CYCl, GAL10*/*CYCl* et *GAL10*/*CYC1* (cf. Figure 3). Le plasmide pCC12 est un plasmide monocopie de levure permettant l'expression de l'ADR mature bovine et de la 3β-HSD bovine sous le contrôle des promoteurs TEF1 et TDH3, respectivement (cf. Figure 4).

Toutes ces souches sont mises à pousser en Erlenmeyer (volume 100 ml et contenant 30 ml de milieu de culture) dans des conditions classiques de fermentation en milieu Kappeli avec comme source de carbone 2% d'éthanol et 0.1 % de glucose. Après 168 heures de culture, 500µl de milieu de culture (cellules + milieu) sont extrait en deux fois à l'aide de 4ml de dichloroéthane. La phase organique est rassemblée puis séparée en deux pour être séchée sous flux d'azote. L'extrait sec est resuspendu dans 100µl d'un mélange acétontrile/eau (50/50 volume/volume) ou 100µl de dichloroéthane. Les extraits resuspendus dans le mélange acétonitrile/eau sont traités par HPLC, ceci permet une analyse de la composition en différents stéroïdes (hydrocortisone, cortexolone, corticosterone, 170H-progesterone, 4 prégnène 17α,20α diol 3 one) comme décrit dans Valvo *et al*., 1994. Les extraits resuspendus dans le dichiloroéthane sont analysés par chromatographie en phase gazeuse comme décrit dans Duport *et al.,* 1998, cette dernière analyse permettant de mesurer la quantité de pregnenolone et de progesterone des échantillons. Pour chacune des souches transformées, on mesure ainsi la productivité en stéroïdes d'une dizaine de clones. Les résultats présentés ci-dessous, donnent les résultats du meilleur des dix clones testés pour chacune des souches UCY24, UCY25, UCY26 et UCY27 transformées par pCV29 et pCC12 et de la souche UCY16 transformée par pFM10.

**Table 2. Bilan des stéroïdes obtenus (résultats exprimés en mg/l sauf la dernière ligne : résultats exprimés en pourcentage de la totalité des stéroïdes)**

| | UCY24 pCV29+ pCC12 | UCY25 pCV29+ pCC12 | UCY26 pCV29+ pCC12 | pCV29+ pCC12 | UCY27 UCY16 UCY4 | pFM10 pCV29+ pCC12 |
|---|---|---|---|---|---|---|
| 17 OH progesterone | 0,6 | 0,6 | 1,0 | 0,6 | 0,5 | 0,2 |
| deoxycorticosterone | 0,5 | 0,0 | 0,2 | 0,3 | 1 | 0,0 |
| cortexolone | 4,5 | 2,7 | 2,1 | 2,0 | 7,6 | 0,7 |
| 4 prégnène 17α, 20α diol 3 one | 0,0 | 0,0 | 0,0 | 0,0 | 0,0 | 4,5 |
| corticostérone | 7,5 | 10,8 | 7,5 | 9,0 | 8,9 | 3,4 |
| hydrocortisone | 17,7 | 29,2 | 22,3 | 24,7 | 15,8 | 12,3 |
| | | | | | | |
| Stéroïdes Totaux | 30,8 | 43,3 | 33,1 | 36,6 | 33,8 | 21,2 |
| % hydrocortisone | 57 | 67 | 67 | 67 | 47 | 58 |

| | | | | | | |
|---|---|---|---|---|---|---|
| NB : On ne détecte ni prégnenolone ni acétate de prégnenolone dans le milieu de culture de ces souches | | | | | | |

Les souches UCY16 pFM10, UCY24 pCV29+pCC12, UCY25 pCV29+pCC12, UCY26 pCV29+pCC12 et UCY27 pCV29+pCC12 présentées ci-dessus sont donc capables de produire des quantités d'hydrocortisone supérieures à celles produites par la souche UCY4/pCV29+pCC12. La quantité totale de stéroïdes passe ainsi de 21,2 µg/ml dans la souche UCY4/pCV29+pCC12 à 43, 3 µg/ml dans la souche UCY25pCV29+pCC12 tandis que la quantité d'hydrocortisone passe de 12,3 µg/ml à 29,2 µg/ml, respectivement. De plus, l'hydrocortisone représente dans ces exemples jusqu'environ 70% des stéroïdes totaux. On observe donc une forte augmentation de la quantité et de la qualité de l'hydrocortisone produite car aucune des ces souches ne produisent le contaminant 4 pregnen 17, 20 diol 3one. Cette dernière caractéristique particulièrement avantageuse est dûe à l'absence des protéines codées par les gènes *GCY1* et/ou *YPR1* qui sont responsables de la réaction de réduction de la cétone en 20.

Il est également intéressant de noter que de manière inattendue, la surproduction maîtrisée de ARH1p sous le contrôle du promoteur *CYC1* augmente de façon significative la quantité de stéroïdes totaux produits ainsi que la production d'hydrocortisone (cf UCY24 pCV29+pCC12 versus UCY25 pCV29+pCC12). Cependant cette expression ne doit pas être trop importante pour obtenir l'effet désiré. Ainsi, si une expression de la protéine ARH1 à un niveau supérieur par rapport à un niveau physiologique est souhaitable, il faut prendre garde à ne pas surexprimer trop fortement cette protéine sous peine de perdre cette augmentation de production en stéroïdes. Ainsi, le promoteur *TEF1* qui est reconnu comme beaucoup plus fort que *CYC1* (Nacken *et al.,* 1996) donne des résultats insatisfaisants (cf. UCY25 pCV29+pCC12 versus UCY27 pCV29+pCC12). En conclusion la souche UCY25/pCV29+pCC12 a un potentiel estimé pour produire 200mg/l d'hydrocortisone en fermenteur avec un seul contaminant majeur (la corticostérone)

### c) Exemple de production en grand volume des souches selon l'invention

Deux des souches précédentes ont été mises à pousser en fermenteur grand volume en milieu Kappeli selon des techniques bien connues de l'homme du métier (cf. par exemple Risenberg D. *et al.,* 1999). La technique de culture utilisée est un discontinu alimenté en milieu concentré (fed-batch). Le fermenteur de volume total 15 litres contient au départ 6 litres de mileu. L'ajout en continu de 4 litres de milieu concentré supplémentaire au cours de la fermentation ainsi que de liquides correcteurs de pH et l'ajout continu d'éthanol amène le volume final à 11,5 litres environ en fin de culture. Les souches UCY4 pCV29+pCC12 et UCY16 pFM10 ont ainsi été cultivées 180h et une production accrue en hydrocortisone a ainsi pu être obtenue (cf. table 3). A partir de ces résultats, il a été extrapolé les résultats envisageables pour les souches UCY24 pCV29+pCC12, UCY25 pCV29+pCC12, UCY26 pCV29+pCC12 et UCY27 pCV29+pCC12 (cf. table 3).

**Table 3 Production d'hydrocortisone de différents clones obtenus selon l'invention (résultats en mg/l)**

| Souches | Hydrocortisone en mg/l |
|---|---|
| UCY4 pCV29+pCC12 | 80 |
| UCY16 pFM10 | 110 |
| UCY24 pCV29+pCC12 | 123* |
| UCY25 pCV29+pCC12 | 203* |
| UCY26 pCV29+pCC12 | 155* |
| UCY27 pCV29+pCC12 | 172* |

| | |
|---|---|
| * Ces résultats ont été obtenus par extrapolation des résultats obtenus en fermenteur grand volume avec les souches UCY4 pCV29+pCC12 et UCY16 pFM10. | |

### Dépôt de matériel biologique

Les organismes suivants ont été déposés le 24 janvier 2001 à la Collection Nationale de Cultures de Microorganismes (CNCM), 25 rue du Docteur Roux, 75724 Paris Cedex 15, France, selon les dispositions du Traité de Budapest.

| | |
|---|---|
| - Souche CDR07 MATα | Numéro d'ordre I-2616 |
| - Souche TGY260 | Numéro d'ordre I-2615 |

### Liste des souches décrites dans la présente demande

**Fy1679-18b** = (n) *MATa ura3-52 trp1-*Δ*63 leu2-*Δ*1 his3-*Δ*200 fenl* GAL fen^{S} ura⁻ trp⁻ leu⁻ his⁻
**Fy1679-28c** = (n) *MATα ura3-52 trp1-*Δ*63 leu2-*Δ*1 his3-*Δ*200 fenl* GAL fen^{S} ura⁻ trp⁻ leu⁻ his⁻
**TGY195** #4 = Fy1679-18b *ypr1 : : URA3*
**TGY212-1** = Fy1679-18b *ypr1*::*TEF1*ₚ::P450c21 [5x?] ura⁻ trp⁻ leu⁻ his⁻
**TGY243-1** = TGY212-1 *gcyl*::*URA3* trp⁻ leu⁻ his⁻
**TGY245-2D** = TGY243-1 *gcyl*::*TDH3*ₚ::P450c21 ura⁻ trp⁻ leu⁻ his⁻
**TGY260-A** = TGY245-2D *LEU2*::*CYC1*p::*ARH1* ura⁻ trp⁻ his⁻
**CDR01** = Fy1679-18b *MATα LEU2*::'b-mat ADR' ura⁻ trp⁻ his⁻
**CDR07** *MATα= MATα ade2*::*GAL10*/*CYClₚ*::Δ⁷ ura⁻ trp⁻ leu⁻ his⁻ ade⁻ (spore de CA10x Fy1679-28c)
**CDR07** *MATa= MATa ade2*::*GAL10*/*CYC1ₚ*::Δ⁷ ura⁻ trp⁻ leu⁻ his⁻ ade⁻ (spore de CA10 x Fy1679-28c)
**CA03** = CDR01 *ade2*::*GAL10*/*CYC1ₚ*::Δ⁷ ura⁻ trp⁻ ade⁻ his⁻, resistant à la nystatine (Duport *et al.,* 1998)
**CA10** = CA03 *erg5*::*PGK1*ₚ*::*hygro^{R} ura⁻ trp⁻ ade⁻ his⁻, resistant à la nystatine et l'hygromycine (Duport *et al.,* 1998)
**SB14** (2n) = TGY260-A x CDR07 Matα
**YCC4** = (n) *MAT*α *ade2*::*GAL10*/*CYCl*ₚ::Δ⁷ *, LEU2::CYC1*ₚ::*ARH1, ypr1*::*TEF1*ₚ::P450c21*, ERG5, fen1*(?), ura⁻ trp⁻ ade⁻ his⁻, resistant à la nystatine, (spore de SB 14)
**YCC8** = YCC4 transformée avec pLIP5 (*TRP1*::*TEF1ₚ*::P450c17::*PGK1ₜ),* ura⁻ ade⁻ his⁻, resistant à la nystatine
**UCY2** = YCC8 transformée avec pTG12093 (*HIS3*::*TDH3*ₚ::CoxVIₚᵣₑ:: matADX::*PGK1* ₜ) : *HIS3*::*TDH3*ₚ:: CoxVIₚᵣₑ::matADX::*PGK1*ₜ, *ERG5, fen1* (?), ura⁻ ade⁻, resistant à la nystatine
**UCY4** = UCY2 *atf2-*Δ::G418^{R} ura⁻ ade⁻, résistante à la nystatine et au G418. **YCC5** = (n) *MAT*α *ade2*::*GAL10*/*CYC1*ₚ::Δ7Reductase, *LEU2*::*CYC1*ₚ::*ARH1, gcyl*::*TDH3*ₚ::P450c21*, ERG5, fen1(?),* ura⁻ trp⁻ ade⁻ his⁻, (resistante à la nystatine, spore de SB 14).
**YCC9** = YCC5 transformée avec pLIP5 (*TRP1*::*TEF1*ₚ::P450c17::*PGK1ₜ),* ura⁻ ade⁻ his⁻, resistant à la nystatine.
**UCY3** = YCC9 transformée avec pTG12093 (*HIS3*::*TDH3*ₚ::CoxVIₚᵣₑ:: matADX::*PGK1* ₜ) : *IHS3*::*TDH3*ₚ:: CoxVIₚᵣₑ::matADX::*PGK1*ₜ, *ERG5, fen1* (?), ura⁻ ade⁻, (resistante à la nystatine).
**UCY26** = UCY3 *atf2*-Δ::G418^{R} ura⁻ ade⁻, (résistante à la nystatine et au G418).
**YSA2** (2n) = TGY245-2D x UCY2.
**UCY5** = (n) *M4r*α *ade2*::*GAL10lCYC1*ₚ::Δ*7Reductase, LEU2*::*CYC1*ₚ::*ARH1, ypr1::TEF1*p::P450c21*, ERG5, gcy1*::*TDH3*ₚ::P450c21 *fen1*(?), ura⁻ trp⁻ his⁻, resistant à la nystatine, (spore de YSA2).
**UCY6** = UCY5 *LEU2*::*TEF1* :: *matADR ::PGK1ₜ,.*
**UCY16** = UCY6, *atf2::URA3::atf2.*
**UCY19** = UCY5, *LEU2::CYC1 :: ARH1.*
**UCY20** = UCY5*, LEU2*::*TEF1*:: *ARH1.*
**UCY25** = UCY19, *atf2*-Δ::G418^{R} (résistante à la nystatine et au G418).
**UCY27** = UCY20, *atf2*-Δ::G418^{R} (résistante à la nystatine et au G418).
**UCY24** = UCY6, *atf2*-Δ::G418^{R} (résistante à la nystatine et au G418).

### Biblioeraphie :

Andersson S. et al., Cloning, structure, and expression of the mitochondrial cytochrome P-450 sterol 26-hydroxylase, a bile acid biosynthetic enzyme. J. Biol. Chem, 1989. 264 (14): p. 8222-8229.
Arreguin de Lorencez M et Kappeli O J, Regulation of gluconeogenic enzymes during the cell cycle of Saccharomyces cerevisiae growing in a chemostat. Gen Microbiol, 1987. 133 (Pt 9): p. 2517-22..
Bonneaud N. et al., A family of low and high copy replicative, integrative and single-stranded S. cerevisiae/E. coli shuttle vectors. Yeast, 1991 Aug-Sep;7(6): p. 609-15
Burgers P.M. et. Percival K.J, Transformation of yeast spheroplasts without cell fusion. Anal Biochem, 1987. 163(2): p. 391-7.
Cauet G. et al., Pregnenolone esterification in Saccharomyces cerevisiae. A potential detoxification mechanism. Eur J Biochem, 1999. 261(1): p. 317-24.
Chua SC. et al., Cloning of cDNA encoding steroid 11 beta-hydroxylase (P450c11). Proc Natl Acad Sci U S A, 1987. Oct;84(20): p. 7193-7.
Degryse E. et al., In vivo cloning by homologous recombination in yeast using a two- plasmid-based system. Yeast, 1995. 11(7): p. 629-40.
Degryse E., In vivo intermolecular recombination in Escherichia coli: application to plasmid constructions. Gene, 1996. 170(1): p. 45-50.
Degryse E. et al., Pregnenolone metabolized to 17alpha-hydroxyprogesterone in yeast: biochemical analysis of a metabolic pathway. J Steroid Biochem Mol Biol, 1999. 71(5-6): p. 239-46.
Dumas B. et al., 11 beta-hydroxylase activity in recombinant yeast mitochondria. In vivo conversion of 11-deoxycortisol to hydrocortisone. Eur J Biochem, 1996. 238(2): p. 495-504.
Duport C. et al., Self-sufficient biosynthesis of pregnenolone and progesterone in engineered yeast. Nat Biotechnol, 1998. 16(2): p. 186-9.
Hu MC. et Chung BC., Expression of human 21-hydroxylase (P450c21) in bacterial and mammalian cells: a system to characterize normal and mutant enzymes. Mol Endocrinol., 1990. 4(6): p. 893-8.
Kawamoto T. et al., Cloning of cDNA and genomic DNA for human cytochrome P-450 11 beta. FEBS Lett, 1990. 269(2): p. 345-9.
Kuronen P. et al., Reversed-phase liquid chromatographic separation and simultaneous profiling of steroidal glycoalkaloids and their aglycones. J Chromatogr A, 1999. 863(1): p. 25-35.
Lacour T., T. Achstetter, et B. Dumas, Characterization of recombinant adrenodoxin reductase homologue (Arh1p) from yeast. Implication in in vitro cytochrome p45011beta monooxygenase system. J Biol Chem, 1998. 273(37): p. 23984-92.
Lathe R. et al., Plasmid and bacteriophage vectors for excision of intact inserts. Gene, 1987. 57 : p. 193-201.
Lecain E. et al., Cloning by metabolic interference in yeast and enzymatic characterization of Arabidopsis thaliana sterol delta 7-reductase. J Biol Chem, 1996. 271(18): p. 10866-73.
Meng-Chun Hu et Bon-chu Chung. Expression of human 21-hydroxylase (P450c21) in bacterial and mammalian cells: A system to characterize normal and mutant enzyme. DNA and Cell Biology. 1991. 10 (3) : p. 201-209.
Nacken, V., T. Achstetter, et E. Degryse, Probing the limits of expression levels by varying promoter strength and plasmid copy number in Saccharomyces cerevisiae. Gene, 1996. 175(1-2): p. 253-60.
Parent SA., et al., Vector systems for the expression, analysis and cloning of DNA sequences in S. cerevisiae. Yeast, 1985. 1(2) : p. 83-138.
Risenberg D. et R. Guthke, High-cell-density cultivation of microorganims. Appl Microbiol Biotechnol, 1999. 51: p. 422-430.
Thierry et al. The complete sequence of the 8.2 kb segment left of MAT on chromosome III reveals five ORFs, including a gene for a yeast ribokinase. Yeast, 1990. Nov-Dec; 6(6): p. 521-34.
Urban, P., et al., Characterization of recombinant plant cinnamate 4-hydroxylase produced in yeast. Kinetic and spectral properties of the major plant P450 of the phenylpropanoid pathway. Eur J Biochem, 1994. 222(3): p. 843-50.
Valvo, L., et al., General high-performance liquid chromatographic procedures for the rapid screening of natural and synthetic corticosteroids. J Pharm Biomed Anal, 1994. 12(6): p. 805-10.
Wu, DA. et al., Expression and functional study of wild-type and mutant human cytochrome P450c21 in Saccharomyces cerevisiae. DNA Cell Biol, 1991. 10(3): p. 201-9.
Yanisch-Perron et al., Improved M13 phage cloning vectors and host strains: nucleotide sequences of the M13mp18 and pUC19 vectors. Gene, 1985. 33(1): p. 103-19.
Zhao HF. et al., Molecular cloning, cDNA structure and predicted amino acid sequence of bovine 3 beta-hydroxy-5-ene steroid dehydrogenase/delta 5-delta 4 isomerase. FEBS Lett, 1989. 259(1): p. 153-7.

## Revendications

1. Souche de levure génétiquement modifiée produisant, de manière autonome à partir d'une source de carbone simple, un stéroïde ou un dérivé de stéroïde, dérivé du métabolisme du cholestérol, ledit stéroïde ou dérivé de stéroïde étant compris dans le groupe constitué de la prégnénolone, la 17α-hydroxy prégnénolone, le cortisol, la cortexolone, la progestérone, la 17α-hydroxy progestérone, et leurs dérivés acétylés, hydroxylés ou portant un dérivé halogéné ou un groupe méthyle, **caractérisée en ce qu'**elle présente une inactivation des gènes endogènes *ATF2, GCY1* et *YPR1.*

2. Souche de levure selon la revendication 1, **caractérisée en ce qu'**elle présente au moins une inactivation additionnelle d'un gène endogène choisi dans le groupe constitué de *ERG5, ARE1, ARE2, ATF1* et *ADE2.*

3. Souche de levure selon la revendication 2, **caractérisée en ce qu'**elle présente une inactivation des gènes endogènes *ERG5, ATF2, GCY1* et *YPR1.*

4. Souche de levure selon l'une des revendications 1 à 3, **caractérisée en ce qu'**elle présente au moins un bloc d'expression pour un gène hétérologue intégré dans le chromosome, à au moins un locus choisi parmi *ADE2, HIS3, TRP1, LEU2, GCY1, ATF2 et YPR1*, l'intégration étant effectuée de manière intragénique ou intergénique, en voisinage immédiat avec ledit locus.

5. Souche de levure selon l'une des revendications 1 à 4, **caractérisée en ce qu'**elle présente au moins un bloc d'expression pour un gène hétérologue situé sur un plasmide multicopie ou un plasmide à bas nombre de copies.

6. Souche de levure selon la revendication 5, **caractérisée en ce que** le plasmide multicopie est choisi parmi les plasmides à base d'un réplicon 2 micron de levure se répliquant dans *Saccharomyces cerevisiae* et que le plasmide à bas nombre de copies est choisi parmi les plasmides basés sur une origine de réplication ARS chromosomique avec un centromère de levure.

7. Souche de levure selon l'une des revendications 1 à 6, **caractérisée en ce qu'**elle présente au moins un gène ou un ADNc hétérologue dans un bloc d'expression, ledit gène ou ADNc étant choisi dans le groupe constitué du gène de la stérol Δ7-réductase, du cytochrome P450 SCC, de l'adrénodoxine, de l'adrénodoxine réductase, de la 3β-hydrostéroïde déshydrogénase isomérase, du cytochrome b5, de la cytochrome P450 réductase, du cytochrome P450 C 17, du cytochrome P450 C21, du cytochrome P450 C11, et des séquences codant pour ces protéines.

8. Souche de levure selon la revendication 7, **caractérisée en ce que** au moins un gène ou ADNc hétérologue est sous le contrôle d'une séquence promotrice choisie dans le groupe constitué des séquences promotrices endogènes de levure *TDH3, TEF1, PGK1, CYC1, GAL10, ATF2, TIR1, ARH1, ADE2,* et du promoteur hybride *GAL10-CYC1.*

9. Souche de levure selon l'une des revendications 7 ou 8, **caractérisée en ce que** la séquence terminatrice d'au moins un gène ou ADNc hétérologue dans le bloc d'expression est choisie parmi les séquences terminatrices des gènes endogènes *PGK1, CYC1, ATF2, ADE2 et NCP1.*

10. Souche de levure selon l'une des revendications 1 à 9, **caractérisée en ce qu'**elle présente le bloc d'expression hétérologue stérol Δ7-réductase intégré dans le chromosome au locus *ADE2.*

11. Souche de levure selon l'une des revendications 1 à 10, **caractérisée en ce qu'**elle comprend au moins une cassette d'expression des gènes codant pour les formes matures P450SCC et de l'adrénodoxine localisée sur un plasmide à grand nombre de copies.

12. Souche de levure selon l'une des revendications 1 à 11, **caractérisée en ce qu'**elle comprend une cassette d'expression de l'adrénodoxine réductase pour P450SCC localisée sur un plasmide mono ou à faible nombre de copies, ou intégrée dans un ou plusieurs chromosomes de levure,

13. Souche de levure selon la revendication 12, **caractérisée en ce que** ladite cassette d'expression de l'adrénodoxine réductase possède les éléments assurant la présence de la protéine dans le cytosol de la cellule hôte.

14. Souche de levure selon l'une des revendications 1 à 13, **caractérisée en ce qu'**elle comprend au moins une cassette d'expression choisie parmi les cassettes d'expression de la 3β-hydrostéroïde déshydrogénase isomérase, du cytochrome P450C 17, du cytochrome P450C21 localisée sur un plasmide à grand nombre de copies.

15. Souche de levure selon l'une des revendications 1 à 14, **caractérisée en ce qu'**elle comprend au moins une cassette d'expression pour la P45011β située sur un plasmide multicopie, la protéine produite présentant un signal d'adressage vers les mitochondries.

16. Souche de levure selon l'une des revendications 1 à 15, **caractérisée en ce qu'**elle comprend au moins une cassette d'expression pour un précurseur de l'adrénodoxine pour P45011β située sur un plasmide multicopie, avec un promoteur faible, la protéine produite présentant un signal d'adressage vers les mitochondries.

17. Souche de levure selon l'une des revendications 1 à 16, **caractérisée en ce qu'**elle présente au moins deux cassettes d'expression pour l'adrénodoxine, de telle sorte qu'une protéine soit active à l'extérieur de la mitochondrie, l'autre étant active dans les mitochondries de la cellule hôte.

18. Souche de levure selon l'une des revendications 1 à 17, **caractérisée en ce qu'**elle comprend au moins une cassette d'expression pour la *NCP1*, l'*ATR1,* et/ou l'*ATR2* située sur un plasmide monocopie ou intégrée dans le chromosome.

19. Souche de levure selon l'une des revendications 1 à 18, **caractérisée en ce qu'**elle exprime la protéine ARH1 à un niveau supérieur par rapport à un niveau physiologique.

20. Souche de levure selon la revendication 19, **caractérisée en ce qu'**elle possède, en plus du gène endogène, une deuxième cassette d'expression pour la protéine ARH1.

21. Souche de levure selon la revendication 20, **caractérisée en ce que** l'expression de la protéine ARH1 est placée sous le contrôle du promoteur *CYC1* dans ladite cassette.

22. Souche de levure selon l'une des revendications 1 à 21, **caractérisée en ce qu'**elle est polyploïde, diploïde, haploïde ou aneuploïde.

23. Souche de levure selon l'une des revendications 1 à 22, **caractérisée en ce qu'**il s'agit d'une souche de *Saccharomyces cerevisiae.*

24. Souche de levure selon la revendication 22, **caractérisée en ce qu'**elle est dérivée de la souche FY 1679-28c ou de la souche FY 1679-18b.

25. Souche de levure selon l'une des revendications 1 à 24, **caractérisée en ce qu'**elle possède les éléments nécessaires pour l'excrétion du stéroïde produit dans le milieu de culture.

26. Procédé de production d'un stéroïde, **caractérisé en ce qu'**il comprend les étapes de fermentation d'une souche de levure selon l'une des revendications 1 à 25 en présence d'une source de carbone simple, et de récupération du stéroïde produit.

27. Préparation pharmaceutique comprenant une souche de levure selon l'une des revendications 1 à 25.

## Claims

1. Genetically modified yeast strain autonomously producing, from a simple carbon source, a steroid or steroid derivative, derived from cholesterol metabolism, said steroid or steroid derivative being included in the group consisting of pregnenolone, 17α-hydroxypregnenolone, cortisol, cortexolone, progesterone, 17α-hydroxyprogesterone, and their acetylated or hydroxylated derivatives or derivatives bearing a halogenated derivative or a methyl group, **characterized in that** it exhibits an inactivation of the endogenous *ATF2, GCY1* and *YPR1* genes.

2. Yeast strain according to Claim 1, **characterized in that** it exhibits at least one additional inactivation of an endogenous gene chosen from the group consisting of *ERG5, ARE1, ARE2, ATF1* and *ADE2*.

3. Yeast strain according to Claim 2, **characterized in that** it exhibits an inactivation of the endogenous *ERG5, ATF2, GCY1* and *YPR1* genes.

4. Yeast strain according to one of Claims 1 to 3, **characterized in that** it has at least one expression block for a heterologous gene integrated into the chromosome, at least one locus chosen from *ADE2, HIS3, TRP1, LEU2, GCY1, ATF2* and *YPR1,* the integration being carried out intragenically or intergenically in the immediate vicinity of said locus.

5. Yeast strain according to one of Claims 1 to 4, **characterized in that** it has at least one expression block for a heterologous gene located on a multicopy plasmid or a low copy plasmid.

6. Yeast strain according to Claim 5, **characterized in that** the multicopy plasmid is chosen from yeast 2-micron replicon-based plasmids which replicate in *Saccharomyces cerevisiae* and **in that** the low copy plasmid is chosen from plasmids based on a chromosomal ARS origin of replication with a yeast centromere.

7. Yeast strain according to one of Claims 1 to 6, **characterized in that** it has at least one heterologous gene or cDNA in a expression block, said gene or cDNA being chosen from the group consisting of the gene of sterol Δ7-reductase, of cytochrome P450 SCC, of adrenodoxin, of adrenodoxin reductase, of 3β-hydrosteroid dehydrogenase isomerase, of cytochrome b5, of cytochrome P450 reductase, of cytochrome P450 C17, of cytochrome P450 C21 and of cytochrome P450 C11, and of the sequences encoding these proteins.

8. Yeast strain according to Claim 7, **characterized in that** at least one heterologous gene or cDNA is under the control of a promoter sequence chosen from the group consisting of the yeast endogenous promoter sequences *TDH3, TEF1, PGK1, CYC1, GAL10, ATF2, TIR1, ARH1* and *ADE2,* and the hybrid promoter *GAL10-CYC1.*

9. Yeast strain according to either of Claims 7 and 8, **characterized in that** the terminator sequence of at least one heterologous gene or cDNA in the expression block is chosen from the terminator sequences of the endogenous genes *PGK1, CYC1, ATF2, ADE2* and *NCP1.*

10. Yeast strain according to one of Claims 1 to 9, **characterized in that** it has the sterol Δ7-reductase heterologous expression block integrated into the chromosome at the *ADE2* locus.

11. Yeast strain according to one of Claims 1 to 10, **characterized in that** it comprises at least one cassette for expression of the genes encoding the mature forms of P450SCC and of adrenodoxin, located on a high copy plasmid.

12. Yeast strain according to one of Claims 1 to 11, **characterized in that** it comprises a cassette for expression of adrenodoxin reductase for P450SCC, located on a single copy plasmid or a low copy plasmid or integrated into one or more yeast chromosomes.

13. Yeast strain according to Claim 12, **characterized in that** said cassette for expression of adrenodoxin reductase has the elements which ensure that the protein is present in the cytosol of the host cell.

14. Yeast strain according to one of Claims 1 to 13, **characterized in that** it comprises at least one expression cassette chosen from the cassettes for expression of 3β-hydrosteroid dehydrogenase isomerase, of cytochrome P450C17 and of cytochrome p450C21, located on a high copy plasmid.

15. Yeast strain according to one of Claims 1 to 14, **characterized in that** it comprises at least one expression cassette for p45011β, located on a multicopy plasmid, the protein produced having a signal for addressing to mitochondria.

16. Yeast strain according to one of Claims 1 to 15, **characterized in that** it comprises at least one expression cassette for a precursor of adrendoxin for P45011β, located on a multicopy plasmid, with a weak promoter, the protein produced having a signal for addressing to mitochondria.

17. Yeast strain according to one of Claims 1 to 16, **characterized in that** it has at least two expression cassettes for adrenodoxin, such that one protein is active outside the mitochondria, the other being active in the mitochondria of the host cell.

18. Yeast strain according to one of Claims 1 to 17, **characterized in that** it comprises at least one expression cassette for *NCP1, ATR1* and/or *ATR2*, located on a single copy plasmid or integrated into the chromosome.

19. Yeast strain according to one of Claims 1 to 18, **characterized in that** it expresses the ARH1 protein at a level higher than a physiological level.

20. Yeast strain according to Claim 19, **characterized in that** it has, in addition to the endogenous gene, a second expression cassette for the ARH1 protein.

21. Yeast strain according to Claim 20, **characterized in that** the expression of the ARH1 protein is placed under the control of the *CYC1* promoter in said cassette.

22. Yeast strain according to one of Claims 1 to 21, **characterized in that** it is polyploid, diploid, haploid or aneuploid.

23. Yeast strain according to one of Claims 1 to 22, **characterized in that** it is a strain of *Saccharomyces cerevisiae*.

24. Yeast strain according to Claim 22, **characterized in that** it is derived from the FY 1679-28c strain or the FY 1679-18b strain.

25. Yeast strain according to one of Claims 1 to 24, **characterized in that** it has the elements required for excreting the steroid produced in the culture medium.

26. Method for producing a steroid, **characterized in that** it comprises the steps of fermenting a yeast strain according to one of Claims 1 to 25 in the presence of a simple carbon source, and of recovering the steroid produced.

27. Pharmaceutical preparation comprising a yeast strain according to one of Claims 1 to 25.

## Patentansprüche

1. Genetisch modifizierter Hefestamm, der autonom ausgehend von einer einfachen Kohlenstoffquelle ein Steroid oder ein Steroidderivat produziert, bei dem es sich um ein Derivat aus dem Cholesterinstoffwechsel handelt, wobei das Steroid oder Steroidderivat aus der Gruppe mit Pregnenolon, 17α-Hydroxypregnenolon, Cortisol, Cortexolon, Progesteron, 17α-Hydroxyprogesteron und deren Acetyl-, Hydroxyderivaten sowie Derivaten, die ein Halogenderivat oder eine Methylgruppe tragen, besteht, **dadurch gekennzeichnet, dass** er eine Inaktivierung der endogenen Gene *ATF2, GCY1* und *YPR1* aufweist.

2. Hefestamm nach Anspruch 1, **dadurch gekennzeichnet, dass** er mindestens eine zusätzliche Inaktivierung eines endogenen Gens aufweist, das aus der Gruppe ausgewählt ist, bestehend aus *ERG5, ARE1, ARE2, ATF1* und *ADE2.*

3. Hefestamm nach Anspruch 2, **dadurch gekennzeichnet, dass** er eine Inaktivierung der endogenen Gene *ERG5, ATF2, GCY1* und *YPR1* aufweist.

4. Hefestamm nach einem der Ansprüche 1 bis 3, **dadurch gekennzeichnet, dass** er mindestens eine Expressionseinheit für ein heterologes Gen, integriert in das Chromosom an mindestens einem Locus, ausgewählt aus *ADE2, HIS3, TRP1, LEU2, GCY1, ATF2* und *YPR1,* aufweist, wobei die Integration intragenisch oder intergenisch in unmittelbarer Nachbarschaft zu diesem Locus erfolgt ist.

5. Hefestamm nach einem der Ansprüche 1 bis 4, **dadurch gekennzeichnet, dass** er mindestens eine Expressionseinheit für ein heterologes Gen aufweist, die sich auf einem Multikopie-Plasmid oder einem Plasmid mit niedriger Kopienzahl befindet.

6. Hefestamm nach Anspruch 5, **dadurch gekennzeichnet, dass** das Multikopie-Plasmid aus Plasmiden auf Basis des Hefe-2-Mikron-Replikons ausgewählt ist, die sich in *Saccharomyces cerevisiae* replizieren, und dass das Plasmid mit niedriger Kopienzahl aus Plasmiden, die auf einem chromosomalen ARS-Replikationsursprung basieren, mit einem Hefe-Zentromer ausgewählt ist.

7. Hefestamm nach einem der Ansprüche 1 bis 6, **dadurch gekennzeichnet, dass** er mindestens ein Gen oder eine heterologe cDNA in einer Expressionseinheit aufweist, wobei das Gen oder die cDNA aus der Gruppe ausgewählt ist, bestehend aus dem Gen für Sterol-Δ7-Reduktase, Cytochrom P450SCC, Adrenodoxin, Adrenodoxin-Reduktase, 3β-Hydrosteroid-Dehydrogenase-Isomerase, Cytochrom b5, Cytochrom-P450-Reduktase, Cytochrom P450 C17, Cytochrom P450 C21, Cytochrom P450 C11 und für diese Proteine kodierenden Sequenzen.

8. Hefestamm nach Anspruch 7, **dadurch gekennzeichnet, dass** mindestens ein Gen oder eine heterologe cDNA unter der Kontrolle einer Promotorsequenz steht, die aus der Gruppe ausgewählt ist, bestehend aus den endogenen Hefe-Promotorsequenzen *TDH3, TEF1, PGK1, CYC1, GAL10, ATF2, TIR1, ARH1, ADE2* und dem Hybridpromotor *GAL10-CYC1.*

9. Hefestamm nach einem der Ansprüche 7 oder 8, **dadurch gekennzeichnet, dass** die Terminatorsequenz mindestens eines Gens oder einer heterologen cDNA in der Expressionseinheit aus den Terminatorsequenzen der endogenen Gene *PGK1, CYC1, ATF2, ADE2* und *NCP1* ausgewählt ist.

10. Hefestamm nach einem der Ansprüche 1 bis 9, **dadurch gekennzeichnet, dass** er eine in das Chromosom am ADE2-Locus integrierte heterologe Expressionseinheit für Sterol-Δ7-Reduktase aufweist.

11. Hefestamm nach einem der Ansprüche 1 bis 10, **dadurch gekennzeichnet, dass** er mindestens eine Expressionskassette für Gene, die für die reifen Formen von P450SCC und Adrenodoxin kodieren, auf einem Plasmid mit hoher Kopienzahl aufweist.

12. Hefestamm nach einem der Ansprüche 1 bis 11, **dadurch gekennzeichnet, dass** er eine Expressionskassette für die Adrenodoxin-Reduktase für P450SCC auf einem Einzelkopie-Plasmid oder einem Plasmid mit niedriger Kopienzahl oder integriert in ein oder mehrere Hefechromosomen aufweist.

13. Hefestamm nach Anspruch 12, **dadurch gekennzeichnet, dass** die Expressionskassette für die Adrenodoxin-Reduktase die Elemente aufweist, die dafür sorgen, dass das Protein im Cytosol der Wirtszelle vorliegt.

14. Hefestamm nach einem der Ansprüche 1 bis 13, **dadurch gekennzeichnet, dass** er mindestens eine Expressionskassette, die aus den Expressionskassetten für 3β-Hydrosteroid-Dehydrogenase-Isomerase, Cytochrom P450 C17, Cytochrom P450 C21 ausgewählt ist, auf einem Plasmid mit hoher Kopienzahl umfasst.

15. Hefestamm nach einem der Ansprüche 1 bis 14, **dadurch gekennzeichnet, dass** er mindestens eine Expressionskassette für P45011β auf einem Multikopie-Plasmid umfasst, wobei das produzierte Protein ein Signal für die Zielsteuerung in Mitochondrien aufweist.

16. Hefestamm nach einem der Ansprüche 1 bis 15, **dadurch gekennzeichnet, dass** er mindestens eine Expressionskassette für einen Adrenodoxin-Vorläufer für P45011β auf einem Multikopie-Plasmid mit einem schwachen Promotor umfasst, wobei das produzierte Protein ein Signal für die Zielsteuerung in Mitochondrien aufweist.

17. Hefestamm nach einem der Ansprüche 1 bis 16, **dadurch gekennzeichnet, dass** er mindestens zwei Expressionskassetten für Adrenodoxin aufweist, derart, dass ein Protein außerhalb der Mitochondrien der Wirtszelle aktiv ist und das andere in den Mitochondrien der Wirtszelle aktiv ist.

18. Hefestamm nach einem der Ansprüche 1 bis 17, **dadurch gekennzeichnet, dass** er mindestens eine Expressionskassette für *NCP1, ATR1* und/oder *ATR2* auf einem Einzelkopie-Plasmid oder integriert in das Chromosom umfasst.

19. Hefestamm nach einem der Ansprüche 1 bis 18, **dadurch gekennzeichnet, dass** er das Protein ARH1 in einem höheren Spiegel im Vergleich zu dem physiologischen Spiegel exprimiert.

20. Hefestamm nach Anspruch 19, **dadurch gekennzeichnet, dass** er zusätzlich zu dem endogenen Gen eine zweite Expressionskassette für das Protein ARH1 besitzt.

21. Hefestamm nach Anspruch 20, **dadurch gekennzeichnet, dass** die Expression des Proteins ARH1 unter der Kontrolle des *CYC1*-Promotors in dieser Kassette steht.

22. Hefestamm nach einem der Ansprüche 1 bis 21, **dadurch gekennzeichnet, dass** er polyploid, diploid, haploid oder aneuploid ist.

23. Hefestamm nach einem der Ansprüche 1 bis 22, **dadurch gekennzeichnet, dass** es sich um einen Stamm von *Saccharomyces cerevisiae* handelt.

24. Hefestamm nach Anspruch 22, **dadurch gekennzeichnet, dass** er ein Derivat des Stamms FY 1679-28c oder des Stamms FY 1679-18b ist.

25. Hefestamm nach einem der Ansprüche 1 bis 24, **dadurch gekennzeichnet, dass** er die notwendigen Elemente für eine Exkretion des produzierten Steroids in das Kulturmedium besitzt.

26. Verfahren zur Herstellung eines Steroids, **dadurch gekennzeichnet, dass** es die Schritte umfasst, in denen man einen Hefestamm nach einem der Ansprüche 1 bis 25 in Gegenwart einer einfachen Kohlenstoffquelle fermentiert und das produzierte Steroid gewinnt.

27. Pharmazeutische Zubereitung, die einen Hefestamm nach einem der Ansprüche 1 bis 25 umfasst.
